# EUROPEAN PATENT SPECIFICATION

(11) **EP 4 026 828 B1**
(45) Date of publication and mention of the grant of the patent: **11.11.2026**
(21) Application number: 20840241.2
(22) Date of filing: 30.04.2020
(51) Int. Cl.: A01K 67/0275, C12N 9/10, C12N 15/63

(54) **AUXIN-INDUCIBLE DEGRON SYSTEM KIT AND USE THEREOF**
AUXININDUZIERBARES DEGRONSYSTEM-KIT UND DESSEN VERWENDUNG
KIT POUR SYSTÈME DE DÉGRON INDUCTIBLE PAR AUXINE, ET APPLICATION ASSOCIÉE

(30) Priority: 16.07.2019 JP 2019131464
(43) Date of publication of application: 13.07.2022
(73) Proprietor: Inter-University Research Institute Corporation Research Organization of Information and Systems, Tokyo 190-8562 (JP); Kake Educational Institution, Okayama-shi, Okayama 700-0005 (JP)
(72) Inventor: KANEMAKI Masato, Mishima-shi, Shizuoka 411-8540 (JP); HAYASHI Ken-ichiro, Okayama-shi, Okayama 700-0005 (JP)
(74) Representative: HGF
(86) International application number: PCT/JP2020/018237
(87) International publication number: WO 2021/009990

(56) References cited:
- WO-A1-2017/029833
- WO-A1-2018/164214
- ZHANG XU ET AL: "AIDing-targeted protein degradation in Drosophila", vol. 284, no. 8, April 2017 (2017-04-01), GB, pages 1178 - 1181, XP093047679, ISSN: 1742-464X, Retrieved from the Internet <URL:https://onlinelibrary.wiley.com/doi/full-xml/10.1111/febs.14054> DOI: 10.1111/febs.14054
- BENCE MELINDA ET AL: "/Cas9-based genome editing for the conditional depletion of endogenous Drosophila melanogaster proteins", THE FEBS JOURNAL, vol. 284, no. 7, 16 April 2017 (2017-04-16), GB, pages 1056 - 1069, XP093047920, ISSN: 1742-464X, Retrieved from the Internet <URL:https://onlinelibrary.wiley.com/doi/full-xml/10.1111/febs.14042> DOI: 10.1111/febs.14042
- A. J. HOLLAND ET AL: "Inducible, reversible system for the rapid and complete degradation of proteins in mammalian cells", PROCEEDINGS OF THE NATIONAL ACADEMY OF SCIENCES, vol. 109, no. 49, 4 December 2012 (2012-12-04), pages E3350 - E3357, XP055347049, ISSN: 0027-8424, DOI: 10.1073/pnas.1216880109
- YAMADA RYOTARO ET AL: "A Super Strong Engineered Auxin-TIR1 Pair", vol. 59, no. 8, August 2018 (2018-08-01), UK, pages 1538 - 1544, XP093047713, ISSN: 0032-0781, Retrieved from the Internet <URL:https://academic.oup.com/pcp/article-pdf/59/8/1538/25501360/pcy127.pdf> DOI: 10.1093/pcp/pcy127
- LAMBRUS BRAMWELL G. ET AL: "Applying the auxin-inducible degradation (AID) system for rapid protein depletion in mammalian cells", BIORXIV, 31 August 2017 (2017-08-31), XP093047909, Retrieved from the Internet <URL:https://www.biorxiv.org/content/10.1101/182840v1.full.pdf> [retrieved on 20230517], DOI: 10.1101/182840
- CAMLIN NICOLE J ET AL: "Auxin-inducible protein degradation as a novel approach for protein depletion and reverse genetic discoveries in mammalian oocytes+", vol. 101, no. 4, 12 July 2019 (2019-07-12), US, pages 704 - 718, XP093047908, ISSN: 0006-3363, Retrieved from the Internet <URL:https://www.ncbi.nlm.nih.gov/pmc/articles/PMC6863966/pdf/ioz113.pdf> DOI: 10.1093/biolre/ioz113
- KOHEI NISHIMURA ET AL: "An auxin-based degron system for the rapid depletion of proteins in nonplant cells", NATURE METHODS, vol. 6, no. 12, 15 December 2009 (2009-12-15), New York, pages 917 - 922, XP055373128, ISSN: 1548-7091, DOI: 10.1038/nmeth.1401
- DATABASE Database CHEMICATS [online] 1 March 2019 (2019-03-01), Database accession no. 0998366508
- NAOYUKI UCHIDA, TAKAHASHI KOJI, IWASAKI RIE, YAMADA RYOTARO, YOSHIMURA MASAHIKO, ENDO TAKAHO A, KIMURA SEISUKE, ZHANG HUA, NOMOTO : "Chemical hijacking of auxin signaling with an engineered auxinÃ Â¢Ã Â Ã Â TIR1 pair", NATURE CHEMICAL BIOLOGY, NATURE PUB. GROUP, NEW YORK, vol. 14, no. 3, New York, pages 299 - 305, XP055555203, ISSN: 1552-4450, DOI: 10.1038/nchembio.2555
- TOYOAKI NATSUME, TOMOMI KIYOMITSU, YUMIKO SAGA, MASATOÃ Â¯Ã Â¿Ã Â½T. KANEMAKI: "Rapid Protein Depletion in Human Cells by Auxin-Inducible Degron Tagging with Short Homology Donors", CELL REPORTS, ELSEVIER INC, US, vol. 15, no. 1, 1 April 2016 (2016-04-01), US, pages 210 - 218, XP055373129, ISSN: 2211-1247, DOI: 10.1016/j.celrep.2016.03.001
- NISHIMURA KOHEI; FUKAGAWA TATSUO: "An efficient method to generate conditional knockout cell lines for essential genes by combination of auxin-inducible degron tag and CRISPR/Cas9", CHROMOSOME RESEARCH, KLUWER ACADEMIC PUBLISHERS, NL, vol. 25, no. 3, 6 June 2017 (2017-06-06), NL, pages 253 - 260, XP036350642, ISSN: 0967-3849, DOI: 10.1007/s10577-017-9559-7
- AISHA YESBOLATOVA, TOYOAKI NATSUME, KENICHIRO HAYASHI, MASATO KANEMAKI: "Chemical and Genetical Improvement of Auxin- Inducible Degron (AID) Technology", ANNUAL MEETING OF THE MOLECULAR BIOLOGY SOCIETY OF JAPAN PROGRAM • ABSTRACTS; NOVEMBER 28-30, 2018, vol. 41, 2018, pages 1P-0747, XP009526233
- NIKKEI BP INC: "piggyBac transposon method", 12 February 2018 (2018-02-12), JP, XP009526232, Retrieved from the Internet <URL:https://bio.nikkeibp.co.jp/atcl/report/16/011900001/18/02/08/00159/>
- YESBOLATOVA AISHA, SAITO YUICHIRO, KANEMAKI MASATO T.: "Constructing Auxin-Inducible Degron Mutants Using an All-in-One Vector", PHARMACEUTICALS, vol. 13, no. 5, pages 103, XP055783487, DOI: 10.3390/ph13050103

## Description

### [Technical Field]

The present invention relates to an auxin-inducible degron system and a target protein degradation method.

Priority is claimed on Japanese Patent Application No. 2019-131464, filed in Japan on July 16, 2019.

### [Background Art]

The present inventors have thus far developed a protein degradation control technology called an auxin-inducible degron system (for example, refer to Patent Literature 1 to 3). In this system, a TIR1 that configures an auxin-responsive ubiquitin ligase is introduced into a cell derived from a eukaryote such as a yeast or an animal cell, and the degradation of a target protein to which a degradation tag (a plant-derived Aux/IAA family protein or a partial protein thereof; also referred to as a degron) has been added is controlled by adjusting the presence or absence or timing of the addition of an auxin.

The method for controlling the degradation of a target protein developed by the present inventors (also referred to as auxin-inducible degron method) has already been broadly used for research in cell biology, and the use of this method for model organisms such as yeasts, nematodes, *Drosophila* and zebrafish is also underway.
Holland et al. describes an inducible, reversible system for the rapid and complete degradation of proteins in mammalian cells. Ryotaro et al. describes a super strong engineered Auxin-TIR1 pair.

### [Citation List]

### [Patent Literature]

[Patent Literature 1]
   Japanese Unexamined Patent Application, First Publication No. 2008-187958
[Patent Literature 2]
   PCT International Publication No. WO 2010/125620
[Patent Literature 3]
   PCT International Publication No. WO 2013/073653

### [Summary of Invention]

### [Technical Problem]

The use of a conventional auxin-inducible degron method makes it possible to rapidly degrade a target protein to which a degradation tag (degron) has been added by the addition of an auxin. However, in the conventional auxin-inducible degron method, even when no auxin is added, weak degradation of a target protein occurs, which makes strict expression control difficult. In addition, a relatively high concentration (100 µM or higher) of an auxin is used for degradation induction, and thus there has been concern over a toxic impact of the auxin particularly on multicellular animals.

The present invention has been made in consideration of the above-described circumstances and provides an auxin-inducible degron system kit enabling strict and full control of protein degradation, a target protein degradation method, a target protein degradation inducer, a cell and a compound.

### [Solution to Problem]

The present invention is defined by the claims.

In one aspect is provided a target protein degradation method using an auxin-inducible degron system that controls degradation of a target protein in a mammalian cell, the method comprising:
culturing the mammalian cell, wherein the mammalian cell comprises
a first nucleic acid that encodes a mutant TIR1 family protein having a mutation at an auxin-binding site;
   and
   a second nucleic acid that encodes a degradation tag including at least a part of an Aux/IAA family protein and
having an affinity to a complex of the mutant TIR1 family protein and an auxin analog,
   and adding an effective concentration of 10 nM to 1uM of the auxin analog to the culture medium,
wherein the mutant TIR1 family protein is a OsTIR1 protein of which an amino acid sequence is at least 80% identical to SEQ ID NO:3 and in which an F74 of the OsTIR1 has mutated into A, G or S,
and wherein the auxin analog is a compound of the following general formula (I-1) or an ester body thereof, 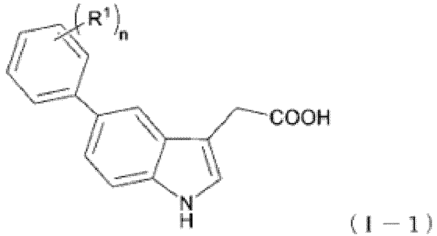
wherein in the general formula (I-1), R1 is an alkyl group having 1 to 6 carbon atoms, an alkoxy group having 1 to 6 carbon atoms, or a halogen atom. n is an integer of 0 to 5, and, in a case where n is an integer of 2, n R1s are identical to or different from each other, and wherein the alkyl group or the alkyl group of the alkoxy group is a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a neopentyl group, a tert-pentyl group, a 2,3-dimethylpropyl group, a 1-ethylpropyl group, a 1-methylbutyl group, a 2-methylbutyl group, an n-hexyl group, an isohexyl group, a 2-methylpentyl group, a 3-methylpentyl group, a 1,1,2-trimethylpropyl group, or a 3,3-dimethylbutyl group.

[1] The description describes an auxin-inducible degron system kit that controls degradation of a target protein in a non-plant-derived eukaryotic cell, the kit containing a first nucleic acid that encodes a mutant TIR1 family protein having a mutation at an auxin-binding site, an auxin analog that has an affinity to the mutant TIR1 family protein, and a second nucleic acid that encodes a degradation tag containing at least a part of an Aux/IAA family protein and having an affinity to a complex of the mutant TIR1 family protein and the auxin analog.
[2] The description describes the kit according to [1], further containing a third nucleic acid that encodes the target protein linked upstream or downstream of the second nucleic acid.
[3] The description describes the kit according to [1] or [2], in which the mutant TIR1 family protein is a rice-derived protein.
[4] The description describes the kit according to any one of [1] to [3], in which the mutant TIR1 family describes protein is a protein in which an F74 of OsTIR1 has mutated into A, G or S.
[5] The description describes the kit according to any one of [1] to [4], in which the auxin analog is a compound represented by the following general formula (1) or an ester body thereof.

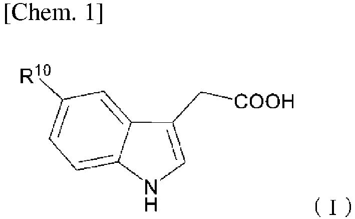

(In the general formula (I), R¹⁰ is a cyclic aliphatic hydrocarbon group in which a substituent may be present and some carbon atoms that configure a ring may be substituted with heteroatoms or an aromatic hydrocarbon group in which a substituent may be present and some carbon atoms that configure a ring may be substituted with heteroatoms.)
The description describes [6] the kit according to any one of [2] to [5], further comprising:
   a fourth nucleic acid that encodes a linker for controlling a plurality of genes with one promoter and that is linked between the first nucleic acid, and the second nucleic acid and the third nucleic acid.
[7] The description describes the kit according to any one of [2] to [6], further containing a transposon vector containing the first nucleic acid and/or the second nucleic acid and the third nucleic acid.
[8] The description describes the kit according to any one of [1] to [7], further containing a target genome DNA cleavage enzyme that encodes an endogenous target protein or a fifth nucleic acid that encodes the enzyme.
[9] The description describes the kit according to any one of [1] to [8], further containing a non-plant-derived eukaryotic cell having the first nucleic acid on a chromosome.
[10] The description describes a target protein degradation method, in which the kit according to any one of [1] to [9] is used.
[11] The description describes a target protein degradation inducer that is used in an auxin-inducible degron system that controls degradation of a target protein in a non-plant-derived eukaryotic cell, the inducer containing a compound represented by the following general formula (1) or an ester body thereof. 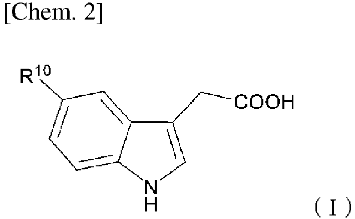 (In the general formula (1), R¹⁰ is a cyclic aliphatic hydrocarbon group in which a substituent may be present and some carbon atoms that configure a ring may be substituted with heteroatoms or an aromatic hydrocarbon group in which a substituent may be present and some carbon atoms that configure a ring may be substituted with heteroatoms.)
[12] The description describes a non-plant-derived eukaryotic cell that is used in an auxin-inducible degron system that controls the degradation of a target protein and has a chromosome containing a first nucleic acid that encodes a mutant TIR1 family protein having a mutation at an auxin-binding site.
[13] The description describes the cell according to [12], in which the mutant TIR1 family protein is a rice-derived protein.
[14] The description describes the cell according to [12] or [13], in which the mutant TIR1 family protein describes is a protein in which an F74 of OsTIR1 has mutated into A, G or S.
[15] The description describes a compound represented by the following general formula (II). 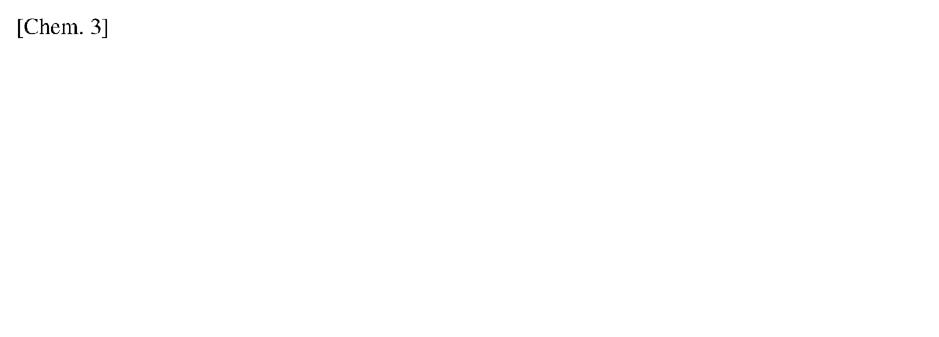 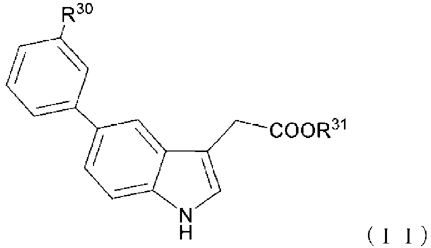 (In the general formula (II), R³⁰ represents a hydrogen atom, an alkyl group having 1 to 6 carbon atoms or a halogen atom and R³¹ represents a hydrogen atom or an alkyl group having 1 to 6 carbon atoms. Here, in a case where R³⁰ is a hydrogen atom or an alkyl group having 1 to 6 carbon atoms, R³¹ is an alkyl group having 1 to 6 carbon atoms.)

### [Advantageous Effects of Invention]

According to the auxin-inducible degron system kit of the present disclosure, strict and full control of protein degradation is possible.

### [Brief Description of Drawings]

Fig. 1 shows the investigation results of degradation of a target protein in an OsTIR1(F74G)/mAID-EGFP-NLS expressing cell in Example 1.
Fig. 2 shows the investigation results of degradation of a target protein in an OsTIR1(F74G)/mAID-EGFP-NLS expressing cell by addition of a plurality of types of auxin analogs in Example 2.
Fig. 3 shows the results of a colony formation test in Example 3.
Fig. 4(A) shows microscopic images of an OsTIR1(F74G)/DHC1-mAID-Clover expressing cell to which 5-Ph-IAA is added. Fig. 4(B) is the confirmation results of 5-Ph-IAA-dependent DHC1 degradation by Western blotting.
Fig. 5 is a conceptual view of a target protein degradation system.
Fig. 6(A) shows the confirmation results of 5-Ph-IAA-dependent CENPH degradation by Western blotting. Fig. 6(B) shows the confirmation results of 5-Ph-IAA-dependent POLD1 degradation by Western blotting.
Fig. 7(A) shows the investigation results of degradation of a target protein in an OsTIR1(WT)/mAID-EGFP-NLS expressing cell and in an OsTIR1(F74G)/mAID-EGFP-NLS expressing cell. Fig. 7(B) shows the FACS analysis results of degradation of a target protein in an OsTIR1(WT)/mAID-EGFP-NLS expressing cell, in an OsTIR1(F74G)/mAID-EGFP-NLS expressing cell and in an OsTIR1(F74A)/mAID-EGFP-NLS expressing cell.
Fig. 8 shows the FACS analysis results of degradation of a target protein in an OsTIR1 (F74G)/mAID-EGFP-NLS expressing cell by addition of a plurality of types of auxin analogs.
Fig. 9(A) shows the investigation results of an influence on the cell cycle of an HCT116 cell treated with 1 µM of 5-Ph-IAA. Fig. 9(B) shows the investigation results of an influence on the colony formation efficiency of the HCT116 cell treated with 1 µM of 5-Ph-IAA.
Fig. 10(A) is a graph showing the results of inducing degradation of an mAID-EGFP-NLS reporter using IAA (auxin) and 5-Ph-IAA in different doses, respectively, in OsTIR1(WT) and in OsTIR1(F74G). Fig. 10(B) is a graph showing the results of inducing degradation of the reporter over time in OsTIR1(WT) and in OsTIR1(F74G).
Fig. 11 shows the investigation results of recovery of mAID-EGFP-NLS expression after removal of 5-Ph-IAA in OsTIR1(F74G).
Fig. 12(A) is a view showing the overview of an experiment in Example 5. Fig. 12(B) is a photograph showing the degree of colony formation on plates in each yeast strain.

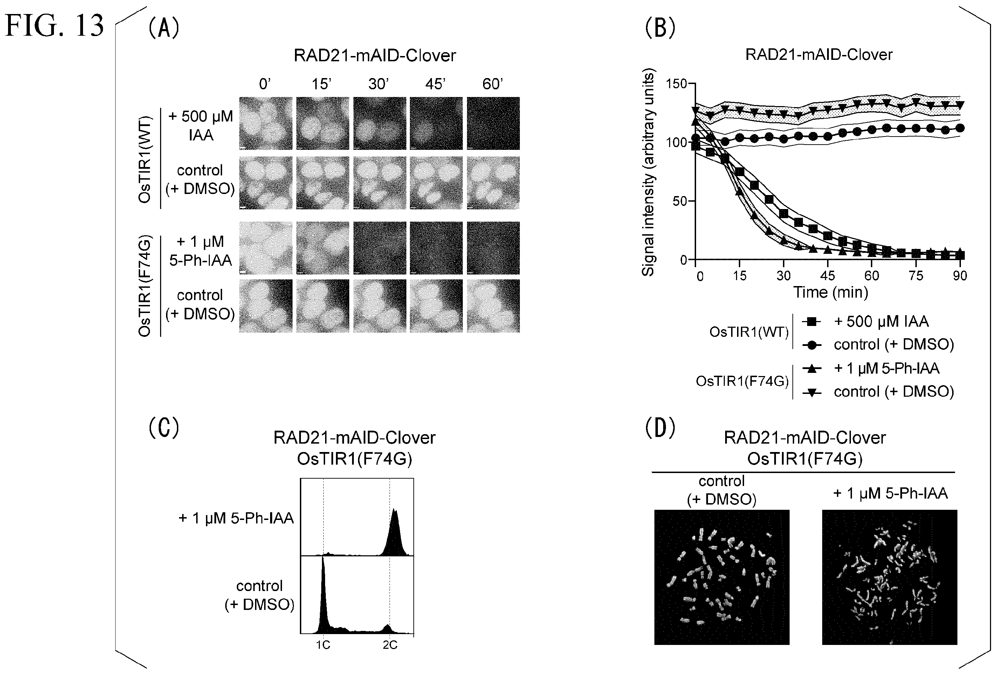

Fig. 13(A) is a photograph of the investigation of degradation of a reporter containing RAD21 in an RAD21-mAID-Clover (RAD21-mAC) cell expressing OsTIR1(WT) or OsTIR1 (F74G). Fig. 13(B) is a graph showing the result of inducing degradation of an RAD21 reporter over time in the RAD21-mAID-Clover (RAD21-mAC) cell expressing OsTIR1(WT) or OsTIR1(F74G). Fig. 13(C) shows the investigation results of an influence of degradation of RAD21 on the cell cycle in the RAD21-mAID-Clover (RAD21-mAC) cell expressing OsTIR1(F74G). Fig. 13(D) is a photograph of the investigation of an influence of the degradation of RAD21 on the agglomeration of sister chromatids in the RAD21-mAID-Clover (RAD21-mAC) cell expressing OsTIR1(F74G).
Fig. 14 is a view showing the overview of an experiment in Example 7.

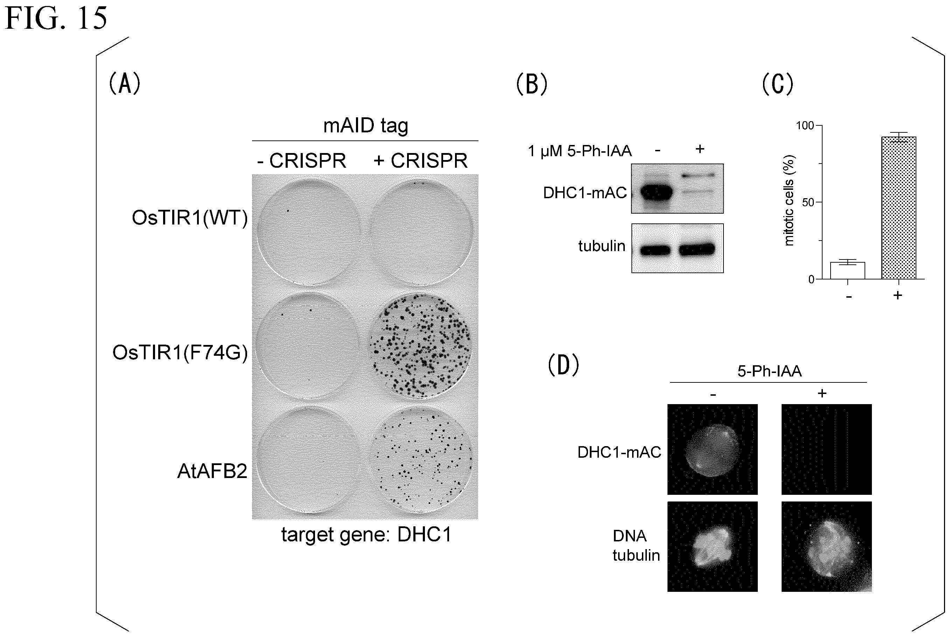

Fig. 15(A) is a photograph showing the presence or absence of colony formation in an OsTIR1(WT or F74G) or AtAFB2 expressing cell in which genome editing has been tried. Fig. 15(B) shows the investigation results of degradation of DHC1-mAC by addition of 1 µM of 5-Ph-IAA in a cell expressing OsTIR1(F74G). Fig. 15(C) is a graph showing the proportion of cells in an M period in a cell expressing OsTIR1(F74G) in which DHC1-mAC is degraded by the addition of 1 µM of 5-Ph-IAA. Fig. 15(D) is a chromatic figure showing the state of a mitotic period in the cell expressing OsTIR1(F74G) in which DHC1-mAC is degraded by the addition of 1 µM of 5-Ph-IAA.
Fig. 16(A) shows alignments of amino acid sequences of mAID and mini-IAA7 (mIAA7). Fig. 16(B) is a photograph showing the presence or absence of colony formation in an OsTIR1(WT or F74G) or AtAFB2 expressing cell in which genome editing is tried using mini-IAA7 (mIAA7). Fig. 16(C) shows the FACS analysis results of degradation of a reporter when degradation of mIAA7 or mAID tag-attached DHC1 is induced in cells expressing AtAFB2 or OsTIR1 (F74G).
Fig. 17 shows the investigation results of degradation of SMC2, CTCF and POLR2A to which mAID is added by addition of 5-Ph-IAA in HCT116 constitutively expressing OsTIR1(F74G).

### [Description of Embodiments]

### <<Auxin-inducible degron system kit>>

A kit of the present disclosure is an auxin-inducible degron system kit that controls degradation of a target protein in a non-plant-derived eukaryotic cell, the kit containing a first nucleic acid that encodes a mutant TIR1 family protein having a mutation at an auxin-binding site, an auxin analog that has an affinity to the mutant TIR1 family protein, and a second nucleic acid that encodes a degradation tag containing at least a part of an Aux/IAA family protein and having an affinity to a complex of the mutant TIR1 family protein and the auxin analog.

An "auxin-inducible degron system" is a protein degradation control technology developed by the present inventors and is a system in which a plant-specific protein degradation system that is introduced by a plant hormone auxin is applied to a non-plant-derived eukaryotic cell (for example, refer to Patent Literature 1 to 3).

Specifically, this system is a system in which a plant-derived TIR1 family protein as an F-box protein, which is a subunit of an E3 ubiquitin ligase complex (SCF complex), and a target protein labeled with a peptide composed of a plant-derived Aux/IAA family protein or a partial sequence thereof are introduced into a non-plant-derived eukaryotic cell, whereby the TIR1 family protein, which is an auxin receptor, recognizes the peptide composed of the Aux/IAA family protein or the partial sequence thereof and degrades the target protein using a ubiquitin/proteasome degradation system in the non-plant-derived eukaryotic cell in an auxin-dependent manner.

In such a system, the present inventors found a problem of the target protein being degraded in an auxin-independent manner. Regarding such a problem, the present inventors found a combination of a mutant TIR1 family protein having a mutation at an auxin-binding site, an auxin analog that has an affinity to the mutant TIR1 family protein and a degradation tag that contains at least a part of an Aux/IAA family protein and has an affinity to a complex of the mutant TIR1 family protein and the auxin analog.

In addition, the present inventors found that the degradation of the target protein can be induced with a low concentration of the auxin analog.

According to the present invention, it is possible to provide an auxin-inducible degron system in which a target protein is hardly degraded when no auxin is added, but the degradation efficiency of the target protein is extremely high when an auxin is added.

### <First nucleic acid>

In the present invention, the first nucleic acid encodes a mutant TIR1 family protein having a mutation at an auxin-binding site.

A TIR1 family protein is an F-box protein, which is a subunit that forms an E3 ubiquitin ligase complex (SCF complex) in protein degradation by an ubiquitin-proteasome system, and a plant-specific protein. The TIR1 family protein serves as a receptor of an auxin, which is a growth hormone, and is known to recognize an Aux/IAA family protein, which is an inhibiting factor of an auxin communication system, by receiving an auxin and to degrade a target protein.

As a gene that encodes the TIR1 family protein, the type thereof is not limited as long as the gene encodes plant-derived TIR1 family proteins. In addition, the type of a plant from which the TIR1 family protein is derived is also not limited, and examples thereof include *Arabidopsis,* rice, zinnia, pines, ferns, *Physcomitrella patens* and the like. Specific examples of the gene that encodes the TIR1 family protein include a TIR1 gene, an AFB1 gene, an AFB2 gene, an AFB3 gene, an FBX14 gene, an AFB5 gene and the like.

Among these, an OsTIR1 gene, which is a rice-derived TIR1 gene, is preferable. As such a gene, genes with an accession number NM_001059194 (GeneID: 4335696) or Os04g0395600 or an accession number EAY93933 or OsI_15707 that are registered in NCBI are exemplified, and more specifically, genes composed of a base sequence represented by the sequence number 1 are exemplified. Furthermore, genes composed of a base sequence represented by the sequence number 2, having codons optimized for human cells, are preferable.

In the present invention, the mutant TIR1 family protein has a mutation at an auxin-binding site. Such a mutant protein is not particularly limited as long as the mutant protein has an affinity to the auxin analog to be described below, but is preferably a protein in which the 74^{th} F of OsTIR1 has mutated into A, G or S and more preferably a protein in which the 74^{th} F of OsTIR1 has mutated into G.

Specifically, the mutant TIR1 family protein is particularly preferably a protein that is composed of sequences including any one of the following amino acid sequences (a) to (c) and is bound with the degradation tag through the complex with the auxin analog to guide the target protein to be degraded.
(a) An amino acid sequence in which the amino acid number 74 site of the amino acid sequence represented by the sequence number 3 is glycine.
(b) An amino acid sequence in which one to several amino acids are deleted, inserted, substituted or added in sites other than the amino acid number 74 site of the amino acid sequence (a).
(c) An amino acid sequence that is 80% or more identical at sites other than the amino acid number 74 site of the amino acid sequence (a).

The number of amino acids deleted, inserted, substituted or added in the amino acid sequence (b) is preferably 1 to 120, more preferably 1 to 60, still more preferably 1 to 20, particularly preferably 1 to 10 and most preferably 1 to 5.

In order to be functionally identical to a protein composed of sequences including the amino acid sequence (a), a protein is 80% or more identical. Regarding such an identity, the protein is more preferably 85% or more identical, still more preferably 90% or more identical, particularly preferably 95% or more identical and most preferably 99% or more identical.

As an F74A protein in OsTIR1, proteins composed of an amino acid sequence represented by the sequence number 4 are exemplified, and, as a gene that encodes the F74A protein in OsTIR1, genes composed of a base sequence represented by the sequence number 5 are exemplified.

As an F74G protein in OsTIR1, proteins composed of an amino acid sequence represented by the sequence number 6 are exemplified, and, as a gene that encodes the F74G protein in OsTIR1, genes composed of a base sequence represented by the sequence number 7 are exemplified.

The first nucleic acid that encodes the mutant TIR1 family protein may be DNA having an exon and an intron or may be cDNA composed of an exon. The first nucleic acid that encodes the mutant TIR1 family protein may be, for example, a full-length sequence in genome DNA or a full-length sequence in cDNA. In addition, the first nucleic acid that encodes the mutant TIR1 family protein may be a partial sequence in genome DNA or a partial sequence in cDNA as long as an expressed protein functions as TIR1.

In the present specification, "functioning as the TIR1 family protein" means that, for example, the expressed protein recognizes a degradation tag (a full-length Aux/IAA family protein or a partial protein) in the presence of the auxin analog. This is because the TIR1 family protein is capable of degrading target proteins labeled with a degradation tag as long as the TIR1 family protein is capable of recognizing the degradation tag.

In the kit of the present disclosure , it is preferable that a promotor sequence that controls the transcription of the first nucleic acid be operably linked to the 5' end of the first nucleic acid that encodes the TIR1 family protein. This makes it possible for the TIR1 family protein to be more reliably expressed.

In the present specification, "being operably linked" means a functional linkage between a gene expression control sequence (for example, a promotor or a series of transcription factor-binding sites) and a gene intended to be expressed (the first nucleic acid that encodes the TIR1 family protein). Here, "expression control sequence" means a sequence that is oriented for the transcription of a gene intended to be expressed (the first nucleic acid that encodes the TIR1 family protein).

The promotor is not particularly limited and can be appropriately determined depending on, for example, the type of a cell or the like. Specific examples of the promotor include a CMV promotor, a SV40 promotor, an EF1a promotor, an RSV promotor and the like.

In the kit of the present disclosure , the first nucleic acid that encodes the TIR1 family protein and the promotor sequence operably linked upstream may be in a form of being inserted into a vector.

The vector is preferably an expression vector. The expression vector is not particularly limited, and an expression vector suitable for the host cell can be used.

In the vector, a polyadenylation signal, an NLS, a marker gene of a fluorescent protein or the like may be operably linked to the 5' end or 3' end of the first nucleic acid that encodes the TIR1 family protein.

The kit of the present disclosure may contain a non-plant-derived eukaryotic cell having the first nucleic acid on a chromosome. Such a cell preferably has the first nucleic acid at a safe harbor site.

"Safe harbor site" means a gene region where constant and stable expression occurs and a region where a gene that is intrinsically encoded to this region can be kept alive even in the case of being defective or altered. In the case of inserting foreign DNA (a gene that encodes TIR1 in the present embodiment) into a safe harbor site using a CRISPR system, a PAM sequence is preferably present in the vicinity thereof. Examples of the safe harbor site include a GTP-binding protein 10 gene locus, a Rosa26 gene locus, a beta-actin gene locus, an AAVS1 (the AAV integration site 1) gene locus and the like. Among them, in the case of a human-derived cell, a foreign DNA is preferably inserted into the AAVS1 gene locus.

The cell is not particularly limited as long as the cell is a non-plant-derived eukaryotic cell, and examples thereof include cells of animals, fungi, protists and the like. Examples of the animals include mammals such as humans, mice, rats and rabbits, fish or amphibians such as zebrafish and *Xenopus laevis* and invertebrates such as *C. elegans* and *Drosophila.*

In addition, examples thereof also include established eukaryote-derived cells, ES cells and iPS cells. Specific examples of the eukaryotic cells include established human-derived cells, established mouse-derived cells, established chicken-derived cells, human ES cells, mouse ES cells, human iPS cells, mouse iPS cells and the like.
Specific examples thereof include human HCT116 cells, human HT1080 cells, human NALM6 cells, human ES cells, human iPS cells, mouse ES cells, mouse iPS cells, chicken DT40 cells and the like.

In addition, examples of the fungi include *Saccharomyces cerevisiae,* fission yeast and the like.

### <Auxin analog>

In the present invention, the auxin analog is not particularly limited as long as the auxin analog has an affinity to the mutant TIR1 family protein, but is preferably a compound represented by the following general formula (I) or an ester body thereof. 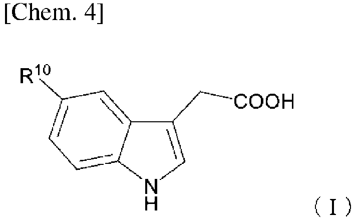 (In the general formula (I), R¹⁰ is a cyclic aliphatic hydrocarbon group in which a substituent may be present and some carbon atoms that configure a ring may be substituted with heteroatoms or an aromatic hydrocarbon group in which a substituent may be present and some carbon atoms that configure a ring may be substituted with heteroatoms.)

### [Cyclic aliphatic hydrocarbon group]

The cyclic aliphatic hydrocarbon group as R¹⁰ may be a monocyclic group or a polycyclic group.

As the monocyclic aliphatic hydrocarbon, a cyclopropyl group, a cyclobutyl group, a cyclopentyl group, a cyclohexyl group, a methylcyclohexyl group, a dimethylcyclohexyl group, a cycloheptyl group, a cyclooctyl group, a cyclononyl group and a cyclodecyl group are exemplified.

As the polycyclic aliphatic hydrocarbon group, a decahydronaphthyl group, an adamantyl group, a 2-alkyladamantan-2-yl group, a 1-(adamantan-1-yl)alkane-1-yl group, a norbornyl group, a methylnorbornyl group, an isobornyl group and the like are exemplified.

In the cyclic aliphatic hydrocarbon group, some carbon atoms that configure the ring may be substituted with heteroatoms. As the heteroatoms, an oxygen atom, a sulfur atom, a nitrogen atom and the like are exemplified. As such a heterocycle, pyrrolidine, tetrahydrofuran, tetrahydrothiophene, piperidine, tetrahydropyran, tetrahydrothiopyran, dioxane, dioxolane and the like are exemplified.

As the substituent, an alkyl group having 1 to 6 carbon atoms, an alkoxy group having 1 to 6 carbon atoms, a halogen atom and an aryl group having 6 to 30 carbon atoms are exemplified.

Examples of the alkyl group include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a neopentyl group, a tert-pentyl group, a cyclopentyl group, a 2,3-dimethylpropyl group, a 1-ethylpropyl group, a 1-methylbutyl group, a 2-methylbutyl group, an n-hexyl group, an isohexyl group, a cyclohexyl group, a 2-methylpentyl group, a 3-methylpentyl group, a 1,1,2-trimethylpropyl group, a 3,3-dimethylbutyl group and the like.

As the alkoxy group, alkoxy groups in which the R portion of -OR is the same as that of the above-described alkyl group having 1 to 6 carbon atoms are exemplified. Among them, as the alkoxy group having the number of carbons, a methoxy group or an ethoxy group is preferable.

Examples of the halogen atom include a fluorine atom, a chlorine atom, a bromine atom, an iodine atom and the like. Among them, a fluorine atom or a chlorine atom is preferable as the halogen atom.

As the aryl group, a phenyl group, a naphthyl group, a benzyl group, a phenethyl group, a biphenyl group, a pentarenyl group, an indenyl group, an anthranyl group, a tetracenyl group, a pentasenyl group, a pyrenyl group, a peryleneyl group, a fluorenyl group and a phenanthryl group are exemplified.

### [Aromatic hydrocarbon group]

As the aromatic hydrocarbon group as R¹⁰, the above-described aryl group having 6 to 30 carbon atoms is exemplified.

In the aromatic hydrocarbon group, some carbon atoms that configure the ring may be substituted with heteroatoms. As the heteroatoms, an oxygen atom, a sulfur atom, a nitrogen atom and the like are exemplified. As such a heterocycle, pyrrole, furan, thiophene, pyridine, imidazole, pyrazole, oxazole, thiazole, pyridazine, pyrimidine, indole, benzimidazole, quinoline, isoquinoline, chromene, isochromene and the like are exemplified.

As the substituent, the same substituents as exemplified in the [Cyclic aliphatic hydrocarbon group] section are exemplified.

The ester body of the compound represented by the general formula (1) is an ester body in which the hydrogen atom in -COOH of the general formula (I) has been substituted with a hydrocarbon group and preferably an ester body in which the hydrogen atom is substituted with an alkyl group.

As such an alkyl group, an alkyl group having 1 to 6 carbon atoms is preferable, and a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a neopentyl group, a tert-pentyl group, a cyclopentyl group, a 2,3-dimethylpropyl group, a 1-ethylpropyl group, a 1-methylbutyl group, a 2-methylbutyl group, an n-hexyl group, an isohexyl group, a cyclohexyl group, a 2-methylpentyl group, a 3-methylpentyl group, a 1,1,2-trimethylpropyl group, a 3,3-dimethylbutyl group and the like are exemplified.

As the compound represented by the general formula (I) in which R¹⁰ is an aromatic hydrocarbon group, a compound represented by the following general formula (I-1) or an ester body thereof is preferable. 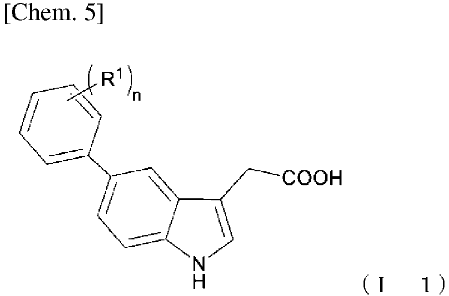 (In the general formula (1-1), R¹ is an alkyl group having 1 to 6 carbon atoms, an alkoxy group having 1 to 6 carbon atoms, a halogen atom or an aryl group having 6 to 30 carbon atoms. n is an integer of 0 to 5, and, in a case where n is an integer of 2 to 5, n R¹'s may be identical to or different from each other.)

Examples of the halogen atom as R¹ include a fluorine atom, a chlorine atom, a bromine atom, an iodine atom and the like. Among them, a fluorine atom or a chlorine atom is preferable as the halogen atom.

Examples of the alkyl group as R¹ include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a neopentyl group, a tert-pentyl group, a cyclopentyl group, a 2,3-dimethylpropyl group, a 1-ethylpropyl group, a 1-methylbutyl group, a 2-methylbutyl group, an n-hexyl group, an isohexyl group, a cyclohexyl group, a 2-methylpentyl group, a 3-methylpentyl group, a 1,1,2-trimethylpropyl group, a 3,3-dimethylbutyl group and the like. Among them, as the alkyl group having 1 to 6 carbon atoms, a methyl group or an ethyl group is preferable.

As the alkoxy group as R¹, alkoxy groups in which the R portion of -OR is the same as that of the above-described alkyl group having 1 to 6 carbon atoms are exemplified. Among them, as the alkoxy group having the number of carbons, a methoxy group or an ethoxy group is preferable.

As the aryl group as R¹, a phenyl group, a naphthyl group, a benzyl group, a phenethyl group, a biphenyl group, a pentarenyl group, an indenyl group, an anthranyl group, a tetracenyl group, a pentasenyl group, a pyrenyl group, a peryleneyl group, a fluorenyl group and a phenanthryl group are exemplified.

n of R¹ is an integer of 0 to 5 and preferably 0 to 3. In a case where the compound represented by the general formula (1-1) has a plurality of R¹'s, the following compound or an ester body thereof is exemplified. 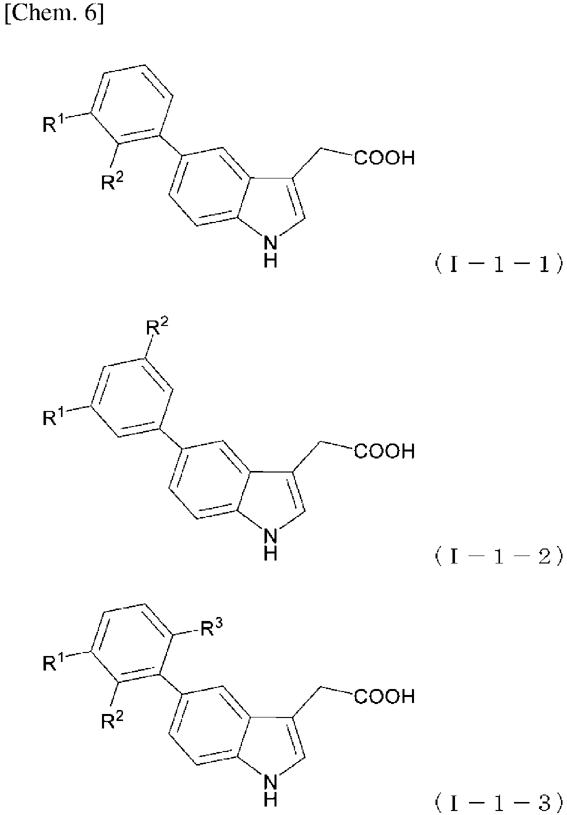 (In general formulae (I-1-1) to (I-1-3), R¹ to R³ are each independently an alkyl group having 1 to 6 carbon atoms, an alkoxy group having 1 to 6 carbon atoms, a halogen atom or an aryl group having 6 to 30 carbon atoms.)

In the general formulae (I-1-1) to (I-1-3), R¹ to R³ are the same as R¹ in the general formula (1-1).

In addition, as the compound represented by the general formula (I), compounds to be described below or ester bodies thereof are preferable.

A compound indicated by the following formula (1-1-4) (also referred to as 5-(3-MeOPh)-IAA).

A compound indicated by the following formula (1-1-5) (also referred to as 5-Ph-IAA).

A compound indicated by the following formula (I-1-6) (also referred to as 5-(3,4-diMePh)-IAA).

A compound indicated by the following formula (1-1-7) (also referred to as 5-(3-MePh)-IAA).

A compound indicated by the following formula (I-1-8) (also referred to as 5-(3-CIPh)-IAA).

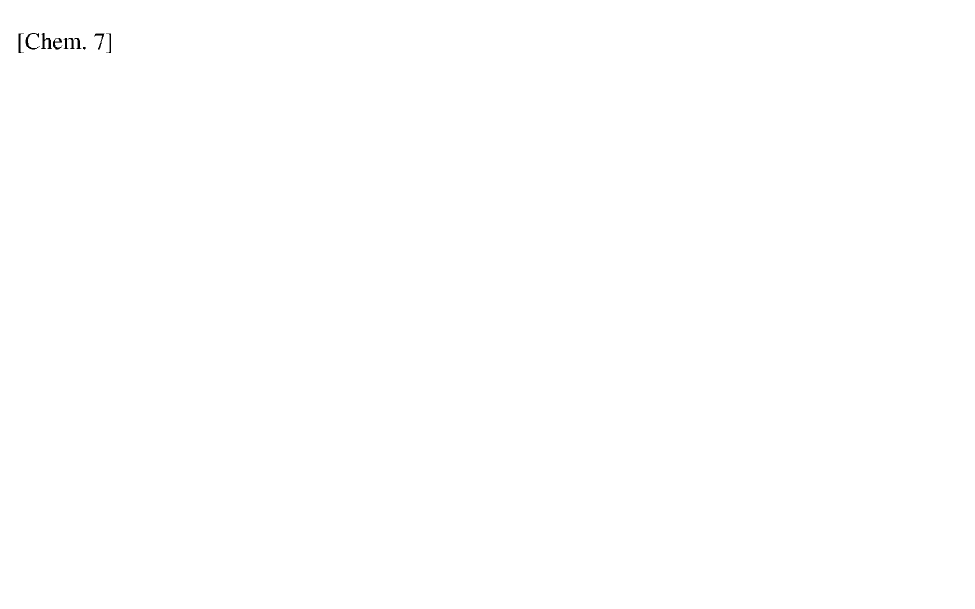

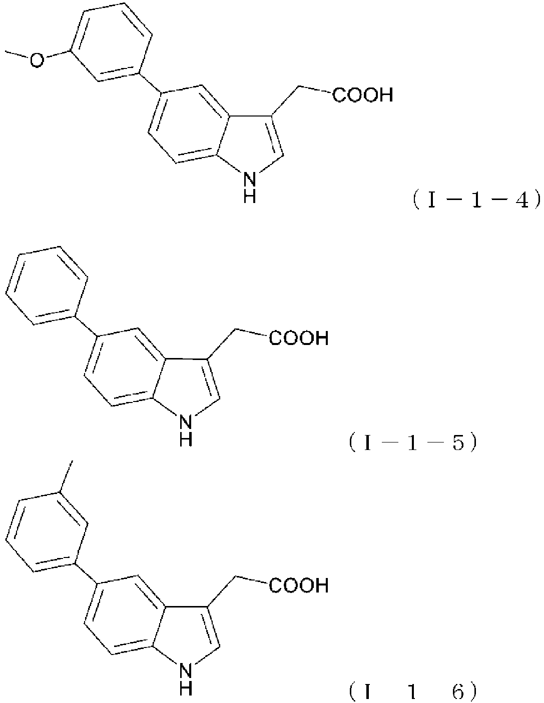

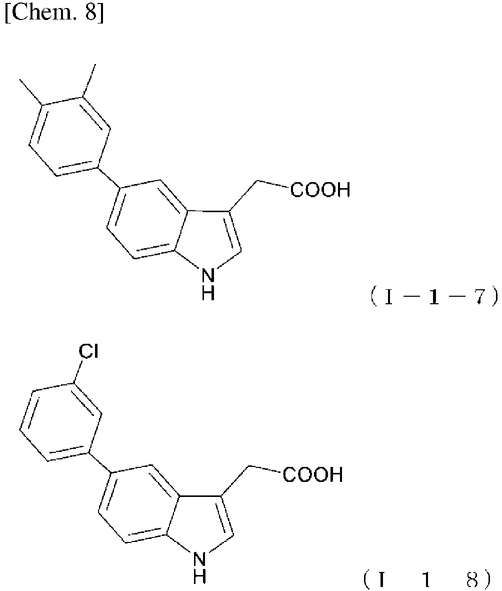

In addition, a compound indicated by the following formula (1-1-9) or an ester body thereof is also preferable.

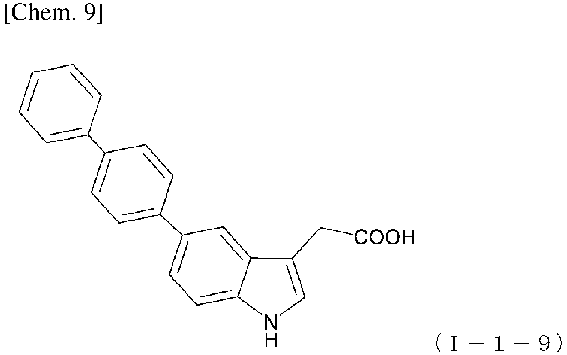

In addition, as the compound in which R¹⁰ is an aromatic hydrocarbon group, compounds represented by the following formulae (I-2) to (I-5) or ester bodies thereof are also preferable.

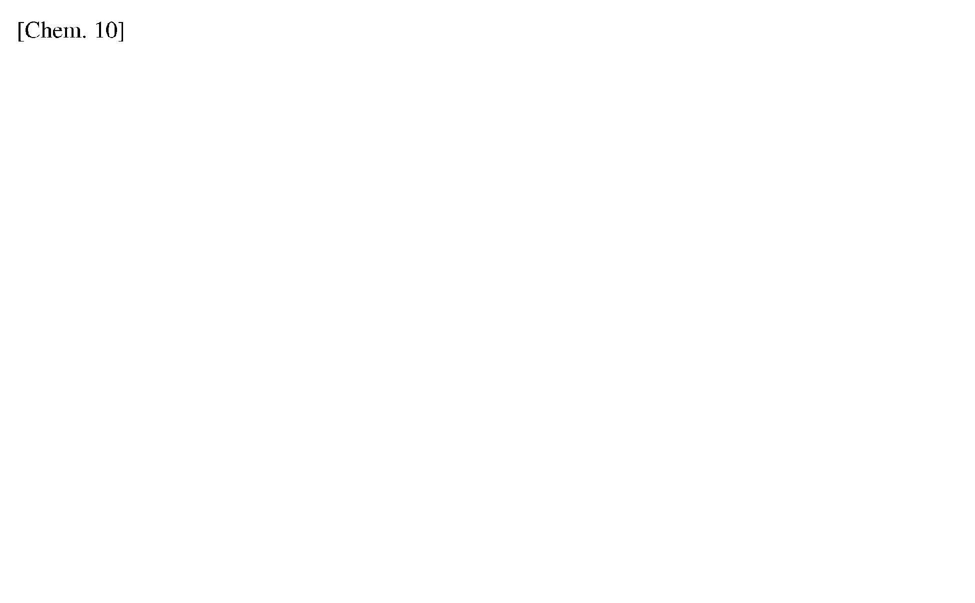

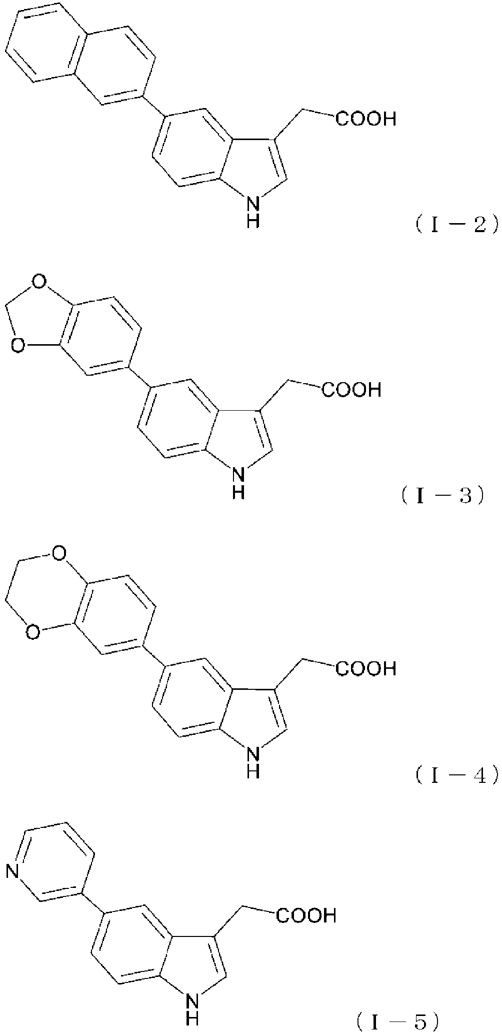

In addition, as the compound in which R¹⁰ is a cyclic aliphatic hydrocarbon group, a compound represented by the following general formula (1-6) or an ester body thereof is also preferable. 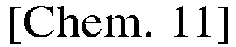 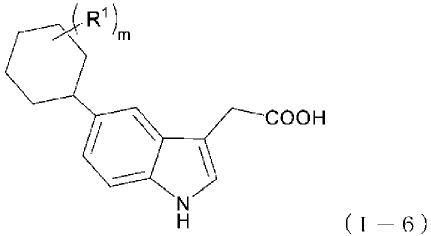 (In the general formula (1-6), R¹ is an alkyl group having 1 to 6 carbon atoms, an alkoxy group having 1 to 6 carbon atoms, a halogen atom or an aryl group having 6 to 30 carbon atoms. m is an integer of 0 to 11, and, in a case where m is an integer of 2 to 11, m R¹'s may be identical to or different from each other.)

R¹ is the same as R¹ exemplified in the general formula (1-1). m is preferably 0 to 6 and more preferably 0 to 3.

In addition, as the compound in which R¹⁰ is a cyclic aliphatic hydrocarbon group, a compound represented by the formula (I-7) or an ester body thereof is also preferable.

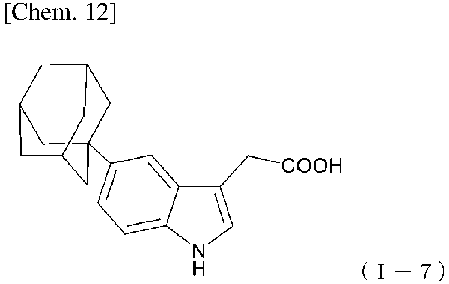

### <Second nucleic acid>

In the present invention, the second nucleic acid encodes a degradation tag containing at least a part of an Aux/IAA family protein and having an affinity to a complex of the mutant TIR1 family protein and the auxin analog.

As a gene that encodes the Aux/IAA family protein, the type thereof is not particularly limited as long as the gene is a plant-derived Aux/IAA family gene.
Specific examples of the gene that encodes the Aux/IAA family protein include an IAA1 gene, an IAA2 gene, an IAA3 gene, an IAA4 gene, an IAA5 gene, an IAA6 gene, an IAA7 gene, an IAA8 gene, an IAA9 gene, an IAA10 gene, an IAA11 gene, an IAA12 gene, an IAA13 gene, an IAA14 gene, an IAA15 gene, an IAA16 gene, an IAA17 gene, an IAA18 gene, an IAA19 gene, an IAA20 gene, an IAA26 gene, an IAA27 gene, an IAA28 gene, an IAA29 gene, an IAA30 gene, an IAA31 gene, an IAA32 gene, an IAA33 gene, an IAA34 gene and the like.

The kit of the present disclosure may have any one type of full-length or partial sequence of the gene that encodes the Aux/IAA family protein or may have two or more types thereof. For example, the sequence of an arabidopsis-derived Aux/IAA family gene is registered in the Arabidopsis Information Resource (TAIR), and the accession number of each gene is as follows.

The IAA1 gene (AT4G14560), the IAA2 gene (AT3G23030), the IAA3 gene (AT1G04240), the IAA4 gene (AT5G43700), the IAA5 gene (AT1G15580), the IAA6 gene (AT1G52830), the IAA7 gene (AT3G23050), the IAA8 gene (AT2G22670), the IAA9 gene (AT5G65670), the IAA10 gene (AT1G04100), the IAA11 gene (AT4G28640), the IAA12 gene (AT1G04550), the IAA13 gene (AT2G33310), the IAA14 gene (AT4G14550), the IAA15 gene (AT1G80390), the IAA16 gene (AT3G04730), the IAA17 gene (AT1G04250), the IAA18 gene (AT1G51950), the IAA19 gene (AT3G15540), the IAA20 gene (AT2G46990), the IAA26 gene (AT3G16500), the IAA27 gene (AT4G29080), the IAA28 gene (AT5G25890), the IAA29 gene (AT4G32280), the IAA30 gene (AT3G62100), the IAA31 gene (AT3G17600), the IAA32 gene (AT2G01200), the IAA33 gene (AT5G57420), the IAA34 gene (AT1G15050).

Among them, the arabidopsis IAA17 gene is preferable.

The degradation tag is not particularly limited as long as the degradation tag is bound with the complex of the mutant TIR1 family protein and the auxin analog and guides the target protein to be degraded, but preferably contains a full-length or partial protein of mAID as the Aux/IAA family protein.

"mAID" is the abbreviation of "mini-auxin-inducible degron" and is a protein composed of a partial sequence of the arabidopsis IAA17, which is one of the Aux/IAA family proteins. This partial sequence is a sequence composed of a region containing at least two Lys residues at each of the N-terminal side and the C-terminal side of a domain II region of the Aux/IAA family protein or a sequence formed by linking two or more sequences described above. This mAID can become a degradation tag that labels the target protein. For example, the amino acid sequence of the mAID is represented by the sequence number 8.

### <Third nucleic acid>

In a case where the target protein has been determined, the kit of the present invention may contain a third nucleic acid that encodes the target protein linked upstream or downstream of the second nucleic acid. The second nucleic acid may be disposed adjacent to any of the 5' side or 3' side of the third nucleic acid.

A fused nucleic acid composed of the second nucleic acid and the third nucleic acid, similar to the first nucleic acid, preferably has a promoter sequence operably linked thereto and may be combined into an expression vector.

### <Fourth nucleic acid>

The first nucleic acid and the second nucleic acid or the fused nucleic acid composed of the second nucleic acid and the third nucleic acid may be each combined into an expression vector, but the kit of the present invention may contain a fourth nucleic acid that encodes a linker for controlling a plurality of genes with one promoter and that is linked between the first nucleic acid, and the second nucleic acid and the third nucleic acid.

As the fourth nucleic acid, a nucleic acid that encodes a read-through linker and a nucleic acid that encodes a non-read-through linker are exemplified.

As the nucleic acid that encodes a read-through linker, a nucleic acid that encodes a cut sequence by an endogenous enzyme is exemplified, and a nucleic acid that encodes a T2A peptide, a nucleic acid that encodes a P2A peptide, a nucleic acid that encodes a F2A peptide and a nucleic acid that encodes an E2A peptide are exemplified.

As the nucleic acid that encodes a non-read-through linker, IRES is exemplified.

### <Transposon vector>

The kit of the present disclosure may contain a transposon vector containing the first nucleic acid and/or the fused nucleic acid composed of the second nucleic acid and the third nucleic acid.

Specifically, the kit of the present disclosure preferably contains a vector containing transposon elements at both ends of the first nucleic acid and/or the fused nucleic acid to which the promotor sequence is operably linked and a vector containing a nucleic acid that encodes transposase.

The first nucleic acid and the fused nucleic acid may be each contained in a separate vector; however, in a case where both are contained in one vector, such a vector preferably contains the fourth nucleic acid.

For the transfer of a transposon, an enzyme that catalyzes a transposition reaction (transposase) and DNA that is recognized by this transposase and transferred (transposon element) are required. The kit of the present disclosure preferably contains such an enzyme and DNA.

In the kit of the present disclosure , in a case where DNA that encodes the transposase and the transposon element are linked together as transposon DNA and combined into one expression system, the transposon DNA is capable of expressing and transferring the transposase on its own once the transposon DNA is introduced into a cell.

Such an autonomous transposon is possibly transferred from a position to which the transposon has been transferred to another position. Therefore, in order to more stably introduce a target gene into a chromosome, the kit of the present disclosure preferably contains DNA that encodes the transposase and the transposon element that are each combined into a separate expression system.

The transposon is not particularly limited, and Sleeping Beauty, piggyBac, Tol 2 and the like are exemplified.

### <Fifth nucleic acid>

The fused nucleic acid composed of the second nucleic acid and the third nucleic acid may be replaced by the endogenous third nucleic acid through homologous recombination; however, in a case where such a fused nucleic acid is combined into an arbitrary chromosome by the transposon or the like, the kit of the present disclosure preferably contains a target genome DNA cleavage enzyme that encodes an endogenous target protein or a fifth nucleic acid that encodes the above-described enzyme. As a system that is used for the double-strand break of a target genome DNA, a CRISPR-Cas9 system, a transcription activator-like effector nuclease (TALEN) system, a Zn finger nuclease system and the like are exemplified. A method for introducing these systems into a cell is not particularly limited, and the target genome DNA cleavage enzyme itself may be introduced into a cell or a target genome DNA cleavage enzyme expression vector containing the fifth nucleic acid may be introduced into a cell.

For example, in the CRISPR-Cas9 system, a method in which a Cas9 expression vector and an expression vector that encodes guide RNA that induces Cas9 in a site that is intended to be cut are introduced into a cell, a method in which an expressed and purified recombinant Cas9 protein and guide RNA are introduced into a cell, or the like is exemplified. The guide RNA may be divided into two parts of tracrRNA and crRNA or may be sgRNA that is connected to one.

### <<Target protein degradation method>>

A target protein degradation method of the present invention is a method in which the above-described kit of the present invention is used. The use of the above-described kit in the auxin-inducible degron system makes strict and full control of target protein degradation possible.

For example, as a method for controlling the degradation of a target protein using the above-described kit, the following method or the like is exemplified.

First, a target protein labeled with a degradation tag and a TIR1 family protein are expressed in a cell. The target protein labeled with a degradation tag and the TIR1 family protein are preferably expressed steadily.

Next, an auxin analog is added to a culture medium. The concentration of the auxin analog that is contained in the culture medium is not limited and is, for example, 1 µM or more and less than 0.1 mM and preferably 10 nM or more and 50 µM or less. As will be described below in examples, in a combination of the compound represented by the general formula (I) and OsTIR1(F74G), it is possible to sufficiently induce degradation of a target protein at a concentration of 50 nM. When a predetermined concentration of an auxin analog is added, a complex of a mutant TIR1 family protein and the auxin analog is formed, this complex recognizes the target protein labeled with a degradation tag, and degradation of the target protein is induced.

According to the degradation method of the present invention, it is possible to induce degradation of a target protein in an auxin analog-specific manner.

### <<Compound>>

A compound of the present invention is a compound represented by the following general formula (11). 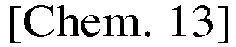 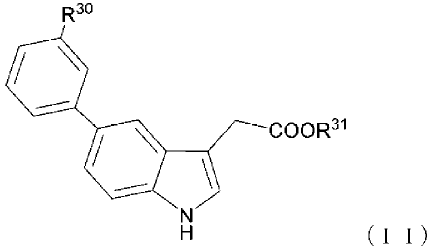 (In the general formula (II), R³⁰ represents a hydrogen atom, an alkyl group having 1 to 6 carbon atoms or a halogen atom and R³¹ represents a hydrogen atom or an alkyl group having 1 to 6 carbon atoms. Here, in a case where R³⁰ is a hydrogen atom or an alkyl group having 1 to 6 carbon atoms, R³¹ is an alkyl group having 1 to 6 carbon atoms.)

Examples of the halogen atom as R³⁰ include a fluorine atom, a chlorine atom, a bromine atom, an iodine atom and the like. Among them, a fluorine atom or a chlorine atom is preferable as the halogen atom.

Examples of the alkyl group as each of R³⁰ and R³¹ independently include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a neopentyl group, a tert-pentyl group, a cyclopentyl group, a 2,3-dimethylpropyl group, a 1-ethylpropyl group, a 1-methylbutyl group, a 2-methylbutyl group, an n-hexyl group, an isohexyl group, a cyclohexyl group, a 2-methylpentyl group, a 3-methylpentyl group, a 1,1,2-trimethylpropyl group, a 3,3-dimethylbutyl group and the like. Among them, as the alkyl group having 1 to 6 carbon atoms, a methyl group or an ethyl group is preferable.

As the compound represented by the general formula (II), compounds represented by the following formulae (II-1) to (II-4) are preferable.

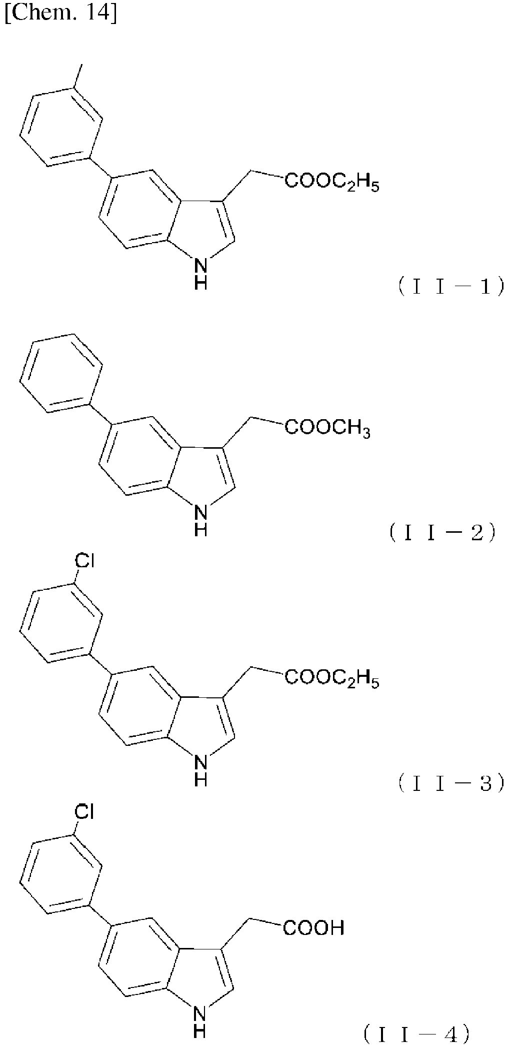

### <<Cell>>

A cell of the present invention is a non-plant-derived eukaryotic cell that is used in auxin-inducible degron systems that control the degradation of target proteins and has a chromosome containing a first nucleic acid that encodes a mutant TIR1 family protein having a mutation at an auxin-binding site.

The cell of the present invention preferably has the first nucleic acid at a safe harbor site.

The cell is the same as that described in the <<Auxin-inducible degron system kit>> section and is not particularly limited as long as the cell is a non-plant-derived eukaryotic cell, and examples thereof include cells of animals, fungi, protists and the like.

The TIR1 family protein is the same as that described in the <<Auxin-inducible degron system kit>> section, and an OsTIR1 gene, which is a rice-derived TIR1 gene, is preferable.

The mutant TIR1 family protein is the same as that described in the <<Auxin-inducible degron system kit>> section, a protein in which the 74^{th} F of OsTIR1 has mutated into A, G or S is preferable, and a protein in which the 74^{th} F of OsTIR1 has mutated into G is more preferable.

Specifically, the mutant TIR1 family protein is the same as that described in the <<Auxin-inducible degron system kit>> section and particularly preferably a protein that is composed of sequences including any one of the above-described amino acid sequences (a) to (c) and is bound with the degradation tag through the complex with the auxin analog to guide the target protein to be degraded.

The cell of the present invention preferably further has a chromosome containing a second nucleic acid that encodes a degradation tag containing at least a part of an Aux/IAA family protein and having an affinity to the complex of the mutant TIR1 family protein and the auxin analog and a third nucleic acid that encodes the target protein linked upstream or downstream of the second nucleic acid.

A method for introducing the first nucleic acid, the second nucleic acid and the third nucleic acid into the chromosomes is not particularly limited, and, as described in the <<Auxin-inducible degron system kit>> section, the nucleic acids may be introduced using a genome-editing technology such as a CRISPR system or may be introduced using a transposon vector.

### [Examples]

Hereinafter, the present invention will be described using examples, but the present invention is not limited to the following examples.

### [Example 1]

### 1. Synthesis of auxin analog

A compound indicated by the formula (I-2) (also referred to as 5-Ph-IAA) was synthesized.

### 2. Preparation of cell

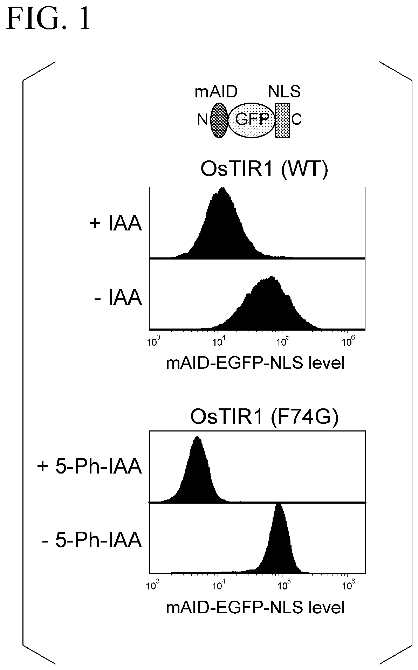

An HCT116 cell (human colon adenocarcinoma-derived cell) having DNA in which an OsTIR1(WT) or OsTIR1(F74G) and an mAID-EGFP (green fluorescent protein)-NLS (nuclear localization signal) were encoded through a P2A sequence inserted onto a chromosome by a transposon (hereinafter, referred to as "OsTIR1(WT)/mAID-EGFP-NLS expressing cell" or "OsTIR1(F74G)/mAID-EGFP-NLS expressing cell") was prepared (refer to Fig. 1).

### 3. Addition of auxin analog

The auxin analog (concentration in culture medium: 0, 200 µM) was added to the OsTIR1(F74G)/mAID-EGFP-NLS expressing cell and cultured for 24 hours. In addition, as a control, a cell containing an auxin added to the OsTIR1(WT)/mAID-EGFP-NLS expressing cell was also prepared.

### 3. FACS analysis

After 24 hours from the addition of the auxin analog, the cells were collected, and FACS analysis was performed. The results are shown in Fig. 1.

Fig. 1 shows that, in the OsTIR1 (F74G)/mAID-EGFP-NLS expressing cell to which the auxin analog was added, expression before degradation induction was high and uniform compared with the OsTIR1(WT)/mAID-EGFP-NLS expressing cell. Furthermore, in the OsTIR1(F74G)/mAID-EGFP-NLS expressing cell to which the auxin analog was added, degradation of GFP was observed more sharply compared with that in the OsTIR1(WT)/mAID-EGFP-NLS expressing cell to which an auxin was added.

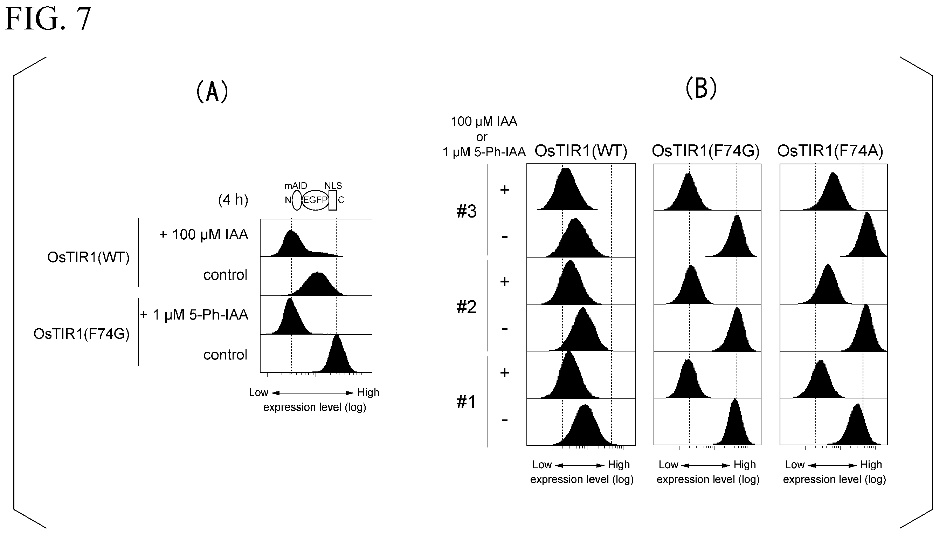

Furthermore, the same experiment was performed by adding 100 µM of an auxin to the OsTIR1(WT)/mAID-EGFP-NLS expressing cell, adding 1 µM of 5-Ph-IAA to the OsTIR1(F74G)/mAID-EGFP-NLS expressing cell, collecting the cells after four hours, and performing FACS analysis. The results are shown in Fig. 7(A).

Comparison of OsTIR1 (WT) and OsTIR1 (F74G) in an isogenic background indicated that, in OsTIR1(WT) with no auxin added thereto, the mAID-EGFP-NLS reporter level was low, the signal peak was wide and basal degradation occurred. It was indicated that the addition of 100 µM of an auxin to OsTIR1(WT) shifted the signal peak to the left and the reporter was degraded.

In contrast, it was indicated that, in the cell expressing OsTIR1(F74G), the expression level of the mAID-EGFP-NLS reporter was high, the signal peak was sharper and, in this cell, basal degradation was low. Furthermore, the addition of 1 µM of 5-Ph-IAA for four hours caused the reporter to be efficiently degraded in the cell expressing OsTIR1(F74G).

Furthermore, the same experiment was also performed on three clones using an OsTIR1(F74A)/mAID-EGFP-NLS expressing cell. The results are shown in Fig. 7(B).

As shown in Fig. 7(B), it was suggested that, in three cell lines expressing OsTIR1 (WT), wider peaks were shown at low expression levels and basal degradation occurred even with no auxin.

On the other hand, it was suggested that, in clones expressing OsTIR1(F74G) and OsTIR1(F74A) with no 5-Ph-IAA added thereto, stronger expression levels of the reporter were shown and basal degradation was suppressed.

Furthermore, in the clones expressing OsTIR1(F74G), sharper degradation of the mAID-EGFP-NLS reporter was shown compared with the clone expressing OsTIR1(F74A) by a 5-Ph-IAA treatment. In all cases, it was shown that the signal peaks shifted to the left after the 5-Ph-IAA treatment and degradation was induced.

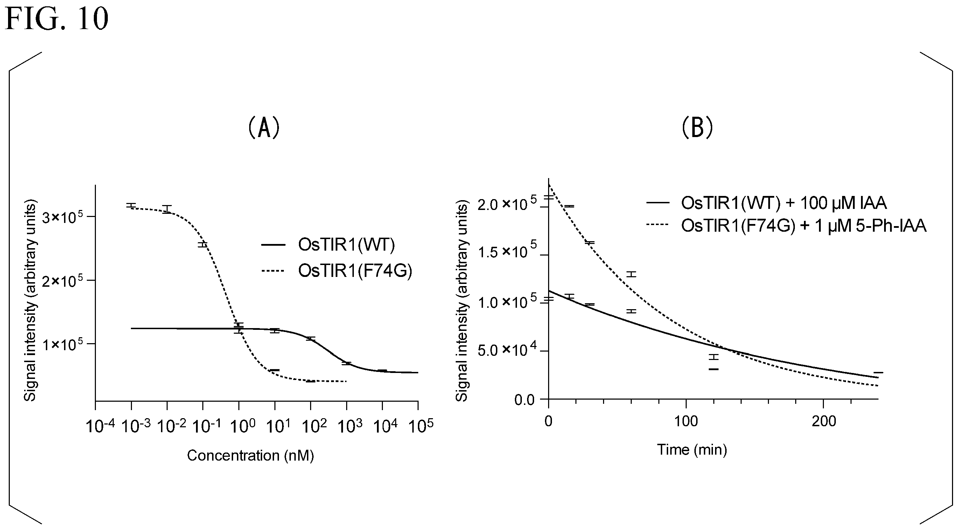

In order to compare OsTIR1(WT) and OsTIR1(F74G) in more detail, the degradation of the mAID-EGFP-NLS reporter was induced using IAA (auxin) and 5-Ph-IAA in different doses, respectively (refer to Fig. 10(A)). As shown in Fig. 10(A), the ligand concentration necessary for the degradation of the cell expressing OsTIR1 (F74G) was significantly low. DC 50 values (concentrations for 50% degradation) were 300 ± 30 nM and 0.45 ± 0.01 nM in OsTIR1(WT) and OsTIR1(F74G), respectively, and it was confirmed that the system in which 5-Ph-IAA was used functioned at an approximately 670-times lower concentration compared with conventional auxin-inducible degron systems.

In order to investigate dynamics over time attributed to the removal of a target protein, samples after a lapse of time were taken, and the expression levels of the reporter were observed (refer to Fig. 10(B)). It was confirmed that the degradation system according to the present invention (also referred to as AID2 system) functioned significantly sharply with a half-life of 62.3 ± 2.0 minutes compared with conventional systems.

On the other hand, in conventional systems, the efficiency decreased with a half-life of 147.1 ± 12.5 minutes. It was confirmed that, in the AID2 system in which OsTIR1 (F74G) was used, the basal degradation of a target protein was small, the concentration of 5-Ph-IAA, which was a new activating ligand, was significantly low, and the degradation rate of the target became fast.

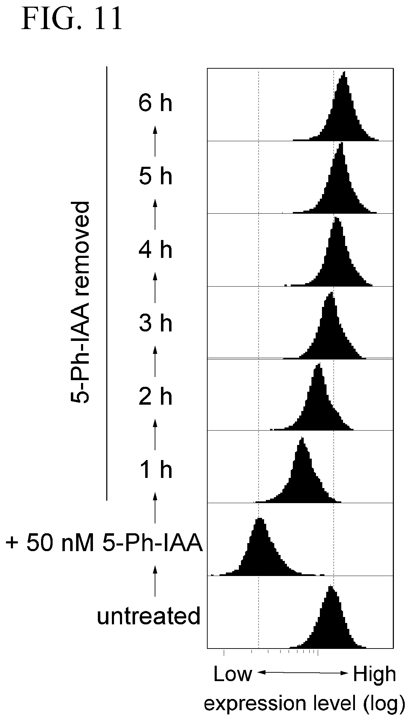

In order to confirm the reversibility of the AID2 system in which a pair of OsTIR1(F74G) and 5-Ph-IAA was used, the degradation of the mAID-EGFP-NLS reporter was induced, then, the culture medium was exchanged with a culture medium containing no 5-Ph-IAA, and the expression of the reporter was investigated. The results are shown in Fig. 11. As shown in Fig. 11, the expression of the reporter was fully recovered after three hours, and the reversibility of the AID2 system was shown.

### [Example 2]

### 1. Synthesis of auxin analogs

A compound indicated by a formula (1-1-5) (also referred to as 5-Ph-IAA), a compound indicated by a formula (1-1-6) (also referred to as 5-(3,4-diMePh)-IAA), a compound indicated by a formula (I-1-7) (also referred to as 5-(3-MePh)-IAA) and a compound indicated by a formula (1-1-8) (also referred to as 5-(3-ClPh)-IAA) were synthesized. The compound indicated by the formula (1-1-4) (also referred to as 5-(3-MeOPh)-IAA) was procured from Tokyo Chemical Industry Co., Ltd.

Hereinafter, a method for synthesizing the synthesized compounds will be described.

### [Synthesis of 5-bromoindole 3-acetic acid methyl ester]

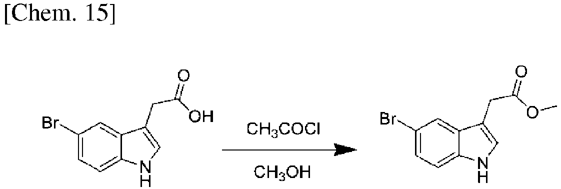

A methanol solution (25 mL) of 5-bromoindole 3-acetic acid (500 mg, 1.97 mmol) was fed into a 100 mL round-bottomed flask, and 1 mL of acetyl chloride was added dropwise thereto under stirring. A reaction was caused at room temperature for three hours. The reaction solution was poured into 100 mL of water, and this was extracted twice with 50 mL of ethyl acetate. An organic layer was washed with 100 mL of saturated salt water, then dehydrated with anhydrous sodium sulfate and condensed under reduced pressure, thereby obtaining 5-bromoindole 3-acetic acid methyl ester of a ginger oil (26 mg, 1.96 mmol, yield: 99%).

The analysis results of the obtained compound by ¹H-NMR and ¹³C-NMR are shown below.

¹H-NMR (400 MHz, CDCl₃) δ 8.17 (s, 1H), 7.73 (d, J = 1.8 Hz, 1H), 7.28 (dd, J = 7.6, 5.7 Hz, 1H), 7.20 (dd, J = 13.5, 4.8 Hz, 1H), 7.16-7.10 (m, 1H), 3.75-3.70 (m, 2H), 3.71 (s, 3H), ¹³C-NMR (100 MHz, CDCl₃) δ C 172.33, 134.80, 129.06, 125.21, 124.42, 121.59, 113.12, 112.730, 108.24, 52.18, 30.98.

### [Synthesis of 5-phenyl-indole-3-acetic acid methyl ester]

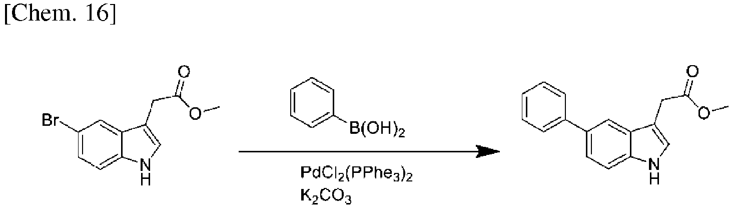

5-Bromoindole 3-acetic acid methyl ester (540 mg, 2.01 mmol), phenylboronic acid (467 mg, 3.83 mmol) and bis(triphenylphosphine)palladium (II) dichloride [PdCl₂(PPhe₃)₂: 67 mg, 0.095 mmol] were added to a 50 mL round-bottomed flask containing 5 mL of dimethylformamide, 5 mL of ethanol and 2 mL of a 3M potassium carbonate aqueous solution. The inside of the container was substituted with nitrogen, and the components were heated and refluxed at 120°C for five hours. 10 mL of ethyl acetate and 20 mL of water were added to the reaction solution, and the reaction solution was extracted three times with 15 mL of ethyl acetate. An organic layer was washed with 20 mL of saturated salt water, subsequently, dehydrated with anhydrous sodium sulfate and then condensed under reduced pressure. This concentrate was purified by silica gel column chromatography (eluting solution, hexane:ethyl acetate = 3:1), and 5-phenyl-indole 3-acetic acid methyl ester was obtained as an orange oil (134 mg, 0.50 mmol, yield: 25.1%)

The analysis results of the obtained compound by ¹H-NMR and ¹³C-NMR are shown below.

¹H-NMR (400 MHz, CDCl₃) δ 8.14 (s, 1H), 7.82 (s, 1H), 7.69-7.61 (m, 2H), 7.43 (t, J = 7.8 Hz, 3H), 7.40-7.34 (m, 1H), 7.34-7.28 (m, 1H), 7.16 (d, J = 10.1 Hz, 1H), 4.17 (qd, J = 7.1, 4.0 Hz, 2H), 3.81 (s, 3H), ¹³C-NMR (100 MHz, CDCl₃) δC 172.19, 142.64, 135.74, 133.37, 128.74, 127.52, 126.44, 123.88, 122.18, 117.53, 111.52, 109.04, 60.95, 31.50.

### [Synthesis of 5-phenyl-indole-3-acetic acid methyl ester (compound indicated by formula (I-1-5) (also referred to as 5-Ph-IAA))]

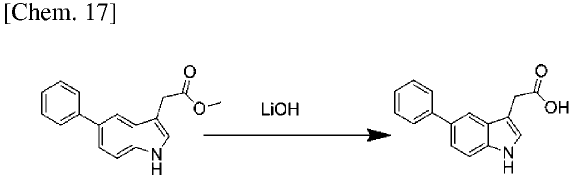

5-Phenyl-indole-3-acetic acid methyl ester (124 mg, 0.47 mmol) was placed in a 10 mL round-bottomed flask, and 1 mL of methanol and 1 mL of tetrahydrofuran were added thereto. A lithium hydroxide aqueous solution (23 mg, 0.96 mmol dissolved in 1 mL of water) was added dropwise to this. This reaction solution was stirred at room temperature for two hours to be hydrolyzed. 5 mL of 1M dilute hydrochloric acid and 5 mL of ethyl acetate were added to the reaction solution to extract a product into an ethyl acetate layer. Furthermore, the product was extracted three times with 5 mL of ethyl acetate. This organic layer was washed with 10 mL of saturated salt water, then dehydrated by adding anhydrous sodium sulfate thereto and condensed under reduced pressure. The concentrate was purified by silica gel column chromatography (chloroform:acetone = 95:5), and 5-phenyl-indole 3-acetic acid was obtained as a caramel powder (98 mg, 0.39 mmol, yield: 83.4%).

The analysis results of the obtained compound by ¹H-NMR and ¹³C-NMR are shown below.

¹H-NMR (400 MHz, acetone-d₆) δ 10.61 (s, 1H), 10.17 (s, 1H), 7.89 (s, 1H), 7.68-7.65 (m, 2H), 7.48-7.46 (m, 1H), 7.44-7.40 (m, 3H), 7.34 (d, J = 2.3Hz, 1H), 7.26 (tt, J = 7.3, 1.4Hz, 1H), 3.82 (s, 2H), ¹³C-NMR (100 MHz, acetone-d₆) δ 173.20, 143.65, 137.15, 133.06, 129.51, 129.15, 127.86, 126.96, 125.39, 121.85, 118.07, 112.54, 109.65, 31.39

### [Synthesis of 5-phenyl-indole-3-acetic acid methyl ester]

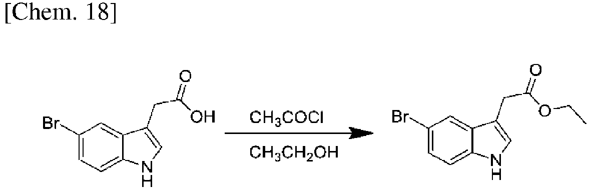

An ethanol solution (25 mL) of 5-bromoindole 3-acetic acid (508 mg, 2.00 mmol) was fed into a 100 mL round-bottomed flask, and 1 mL of acetyl chloride was added dropwise to this under stirring. This reaction solution was stirred at room temperature for three hours. The reaction solution was poured into 100 mL of water, and this was extracted twice with 50 mL of ethyl acetate. An organic layer was washed with 100 mL of saturated salt water, then dehydrated with anhydrous sodium sulfate and condensed under reduced pressure, thereby obtaining 5-bromoindole 3-acetic acid ethyl ester of a ginger oil (556 mg, 1.97 mmol, yield: 99%).

The analysis results of the obtained compound by ¹H-NMR and ¹³C-NMR are shown below.

¹H-NMR (400 MHz, CDCl₃) δ 8.23 (s, 1H), 7.73 (s, 1H), 7.24 (dd, J = 8.5, 2.1 Hz, 1H), 7.15 (d, J = 8.7 Hz, 1H), 7.07 (s, 1H), 4.18 (q, J = 7.2 Hz, 2H), 3.71 (s, 2H), 1.28 (t, J = 7.3 Hz, 3H), ¹³C-NMR (100 MHz, CDCl₃) δ 172.07, 134.82, 129.05, 125.08, 124.49, 121.64, 112.99, 112.77, 108.21, 61.11, 31.31, 14.32.

### [Synthesis of 5-(3-methylphenyl)-indole-3-acetic acid ethyl ester]

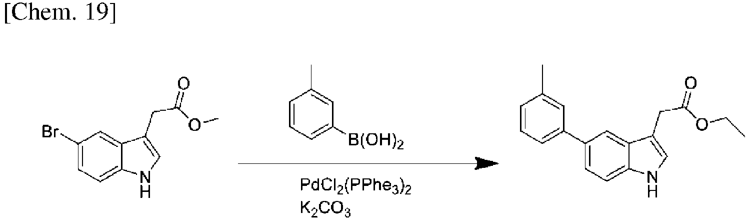

5-Bromoindole 3-acetic acid methyl ester (112 mg, 0.40 mmol), 3-methylphenylboronic acid (107 mg, 0.79 mmol) and bis(triphenylphosphine)palladium (II) dichloride (PdCl₂(PPhe₃)₂: 15 mg, 0.02 mmol) were added to a 50 mL round-bottomed flask containing 1 mL of dimethylformamide, 1 mL of ethanol and 0.5 mL of a 3M potassium carbonate aqueous solution. The inside of the container was substituted with nitrogen, and the components were heated and refluxed at 120°C for five hours. 10 mL of ethyl acetate and 20 mL of water were added to the reaction solution, and the reaction solution was extracted three times with 15 mL of ethyl acetate. An organic layer was washed with 20 mL of saturated salt water, subsequently, dehydrated with anhydrous sodium sulfate and then condensed under reduced pressure. This concentrate was purified by silica gel column chromatography (eluting solution, hexane:ethyl acetate = 3:1), and 5-(3-methylphenyl)-indole 3-acetic acid ethyl ester was obtained as a yellow oil (17 mg, 0.058 mmol, yield: 14.6%).

The analysis results of the obtained compound by ¹H-NMR and ¹³C-NMR are shown below.

¹H-NMR (400 MHz, CDCl₃) δ 8.10 (s, 1H), 7.81 (s, 1H), 7.49-7.42 (m, 3H), 7.39 (d, J = 8.2Hz, 1H), 7.32 (t, J = 7.8, 1H), 7.19 (d, J = 1.1 Hz, 1H), 7.16-7.11 (m, 1H), 4.18 (q, J = 7.6, 2H), 3.82 (s, 2H), 2.41 (s, 3H), 1.27 (t, J = 7.1 Hz, 3H), ¹³C-NMR (100 MHz, CDCl₃) δC 172.12, 142.59, 138.23, 135.68, 133.51, 128.62, 128.32, 127.84, 127.19, 124.61, 123.74, 122.24, 117.51, 111.39, 109.13, 60.91, 31.49, 21.67, 14.34.

### [Synthesis of 5-(3-methylphenyl)-indole-3-acetic acid]

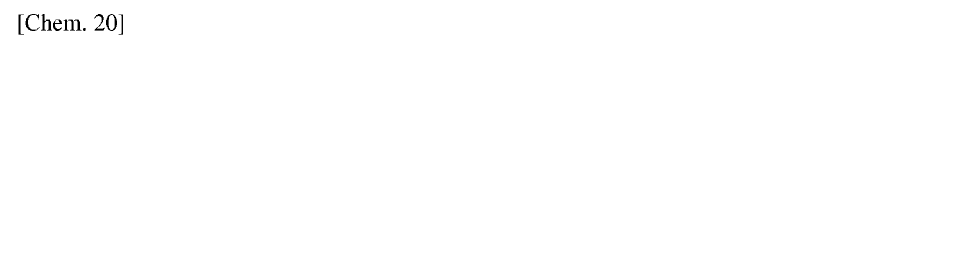

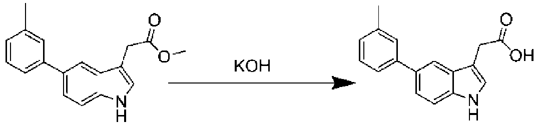

5-(3-Methylphenyl)-indole-3-acetic acid ethyl ester (40 mg, 0.14 mmol) was placed in a 5 mL glass vial, and 0.25 mL of methanol and 0.25 mL of tetrahydrofuran were added thereto. 0.25 mL of a 10% (W/V) potassium hydroxide aqueous solution was added dropwise to this. This reaction solution was stirred at room temperature for two hours to be hydrolyzed. 1 mL of 1M dilute hydrochloric acid and 2 mL of ethyl acetate were added to the reaction solution, and the reaction solution was extracted. Furthermore, the reaction solution was extracted three times with 2 mL of ethyl acetate. This organic layer was washed with 1 mL of saturated salt water, then dehydrated by adding anhydrous sodium sulfate thereto and condensed under reduced pressure. The concentrate was purified by silica gel column chromatography (chloroform:acetone = 95:5), and 5-(3-methylphenyl)-indole 3-acetic acid was obtained as a caramel powder (29 mg, 0.11 mmol, yield: 80.2%). The analysis results of the obtained compound by ¹H-NMR and ¹³C-NMR are shown below.

¹H-NMR (400 MHz, CDCl₃) δ 8.05 (s, 1H), 7.77 (d, J = 1.4 Hz, 1H), 7.48-7.40 (m, 3H), 7.36 (d, J = 8.7 Hz, 1H), 7.30 (t, J = 7.3 Hz, 1H), 7.16-7.09 (m, 2H), 3.82 (s, 2H), 2.41 (s, 3H), ¹³C-NMR (100 MHz, CDCl₃) δC 177.84, 142.51, 138.29, 135.63, 133.70, 128.67, 128.36, 127.69, 127.27, 124.68, 124.04, 122.41, 117.34, 111.53, 108.22, 31.07, 21.68.

### [Synthesis of 5-(3,4-dimethylphenyl)-indole-3-acetic acid ethyl ester]

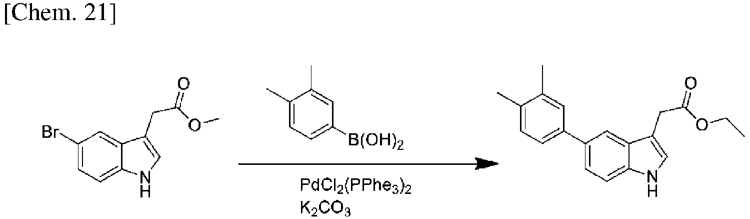

5-Bromoindole 3-acetic acid methyl ester (113 mg, 0.40 mmol), 3,4-dimethylphenylboronic acid (120 mg, 0.80 mmol) and bis(triphenylphosphine)palladium (11) dichloride (PdCl₂(PPhe₃)₂: 15 mg, 0.02 mmol) were added to a 50 mL round-bottomed flask containing 1 mL of dimethylformamide, 1 mL of ethanol and 0.5 mL of a 3M potassium carbonate aqueous solution. The inside of the container was substituted with nitrogen, and the components were heated and refluxed at 120°C for five hours. 10 mL of ethyl acetate and 20 mL of water were added to the reaction solution, and the reaction solution was extracted three times with 15 mL of ethyl acetate. An organic layer was washed with 20 mL of saturated salt water, subsequently, dehydrated with anhydrous sodium sulfate and then condensed under reduced pressure. This concentrate was purified by silica gel column chromatography (eluting solution, hexane:ethyl acetate = 3:1), and 5-(3,4-dimethylphenyl)-indole 3-acetic acid ethyl ester was obtained as a yellow oil (36 mg, 0.12 mmol, yield: 29.2%).

The analysis results of the obtained compound by ¹H-NMR and ¹³C-NMR are shown below.

¹H-NMR (400 MHz, CDCl₃) δ 8.10 (s, 1H), 7.79 (d, J = 0.9 Hz, 1H), 7.44 (m, 2H), 7.38 (m, 2H), 7.22-7.14 (m, 2H), 4.17 (q, J = 7.0 Hz, 2H), 3.80 (s, 2H), 2.35 (s, 3H), 2.31 (s, 3H), 1.25 (t, J = 7.0 Hz, 3H), ¹³C-NMR (100 MHz CDCl₃) δC 172.17, 140.24, 136.80, 135.55, 134.75, 133.44, 130.30, 128.80, 127.83, 124.85, 123.70, 122.15, 117.23, 111.37, 109.04, 60.89, 31.49, 20.04, 19.46, 14.35

### [Synthesis of 5-(3,4-dimethylphenyl)-indole-3-acetic acid]

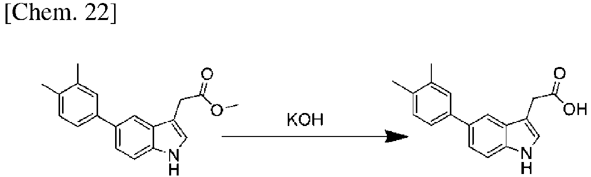

5-(3,4-Dimethylphenyl)-indole-3-acetic acid ethyl ester (30 mg, 0.098 mmol) was placed in a 5 mL glass vial, and 0.25 mL of methanol and 0.25 mL of tetrahydrofuran were added thereto. 0.25 mL of a 10% (W/V) potassium hydroxide aqueous solution was added dropwise to this. This reaction solution was stirred at room temperature for two hours to be hydrolyzed. 1 mL of 1M dilute hydrochloric acid and 2 mL of ethyl acetate were added to the reaction solution, and the reaction solution was extracted. Furthermore, the reaction solution was extracted three times with 2 mL of ethyl acetate. This organic layer was washed with 1 mL of saturated salt water, then dehydrated by adding anhydrous sodium sulfate thereto and condensed under reduced pressure. The concentrate was purified by silica gel column chromatography (chloroform:acetone = 95:5), and 5-(3,4-dimethylphenyl)-indole 3-acetic acid was obtained as a caramel powder (20 mg, 0.0716 mmol, yield: 73.4%).

The analysis results of the obtained compound by ¹H-NMR and ¹³C-NMR are shown below.

¹H-NMR (400 MHz, CDCl₃) δ 8.06 (s, 1H), 7.76 (s, 1H), 7.46-7.40 (m, 2H), 7.37 (d, J = 8.2Hz, 2H), 7.22-7.14 (m, 2H), 3.84 (s, 2H), 2.33 (s, 3H), 2.30 (s, 3H), ¹³C-NMR (100 MHz, CDCl₃) δC 177.32, 140.13, 136.83, 135.50, 134.83, 133.67, 130.05, 128.83, 127.69, 124.89, 123.91, 122.34, 117.10, 111.45, 108.23, 30.99, 20.03, 19.46.

### [Synthesis of 5-(3-chlorophenyl)-indole-3-acetic acid ethyl ester]

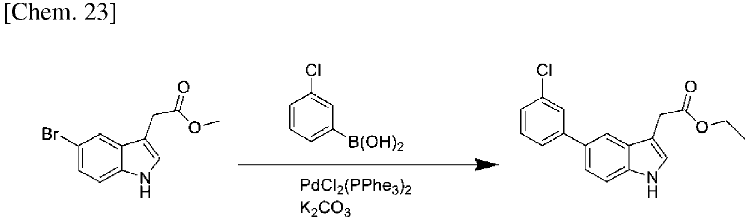

5-Bromoindole 3-acetic acid methyl ester (113 mg, 0.40 mmol), 3-chlorophenylboronic acid (128 mg, 0.82 mmol) and bis(triphenylphosphine)palladium (11) dichloride (PdCl₂(PPhe₃)₂: 15 mg, 0.02 mmol) were added to a 50 mL round-bottomed flask containing 1 mL of dimethylformamide, 1 mL of ethanol and 0.5 mL of a 3M potassium carbonate aqueous solution. The inside of the container was substituted with nitrogen, and the components were heated and refluxed at 120°C for five hours. 10 mL of ethyl acetate and 20 mL of water were added to the reaction solution, and the reaction solution was extracted three times with 15 mL of ethyl acetate. An organic layer was washed with 20 mL of saturated salt water, subsequently, dehydrated with anhydrous sodium sulfate and then condensed under reduced pressure. This concentrate was purified by silica gel column chromatography (eluting solution, hexane:ethyl acetate = 3:1), and 5-(3-chlorophenyl)-indole 3-acetic acid ethyl ester was obtained as a yellow oil (25 mg, 0.79 mmol, yield: 19.5%).

The analysis results of the obtained compound by ¹H-NMR and ¹³C-NMR are shown below.

¹H-NMR (400 MHz, CDCl₃) δ 8.14 (s, 1H), 7.80 (s, 1H), 7.63 (t, J = 1.8 Hz, 1H), 7.53 (dt, J = 8.0, 1.4 Hz, 1H), 7.42 (d, J = 1.4 Hz, 2H), 7.36 (t, J = 8.0 Hz, 1H), 7.30-7.24 (m, 1H), 7.23 (d, J = 2.3 Hz, 1H), 4.18 (q, J = 7.2 Hz, 2H), 3.81 (s, 2H), 1.27 (t, 7.2 Hz, 3H), ¹³C-NMR (100 MHz, CDCl₃) δC 171.99, 144.48, 135.96, 134.54, 131.96, 129.90, 127.98, 127.52, 126.38, 125.59, 123.99, 121.98, 117.68, 111.61, 109.29, 60.95, 31.45, 14.34.

### [Synthesis of 5-(3-chlorophenyl)-indole-3-acetic acid (compound indicated by formula (I-1-8) (also referred to as 5-(3-ClPh)-IAA))]

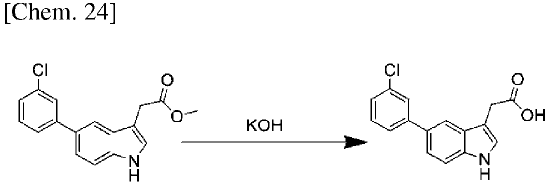

5-(3-Chlorophenyl)-indole-3-acetic acid ethyl ester (38 mg, 0.12 mmol) was placed in a 5 mL glass vial, and 0.25 mL of methanol and 0.25 mL of tetrahydrofuran were added thereto. 0.25 mL of a 10% (W/V) potassium hydroxide aqueous solution was added dropwise to this. This reaction solution was stirred at room temperature for two hours to be hydrolyzed. 1 mL of 1M dilute hydrochloric acid and 2 mL of ethyl acetate were added to the reaction solution, and the reaction solution was extracted. Furthermore, the reaction solution was extracted three times with 2 mL of ethyl acetate. This organic layer was washed with 1 mL of saturated salt water, then dehydrated by adding anhydrous sodium sulfate thereto and condensed under reduced pressure. The concentrate was purified by silica gel column chromatography (chloroform:acetone = 95:5), and 5-(3-chlorophenyl)-indole 3-acetic acid was obtained as a caramel powder (31 mg, 0.11 mmol, yield: 89.6%).

The analysis results of the obtained compound by ¹H-NMR and ¹³C-NMR are shown below.

¹H-NMR (400 MHz, CDCl₃) δ 8.13 (s, 1H), 7.76 (s, 1H), 7.61 (t, J = 1.8Hz, 1H), 7.49 (dt, J = 7.8, 1.4 Hz, 1H), 7.40 (s, 2H), 7.34 (t, J = 7.8 Hz, 1H), 7.26-7.28 (m, 1H), 7.20 (d, J = 2.3 Hz, 1H), 3.82 (s, 2H), ¹³C-NMR (100 MHz, CDCl₃) δC 177.42, 144.36, 135.92, 134.54, 132.15, 129.94, 127.74, 127.54, 126.45, 125.66, 124.26, 122.15, 117.51, 111.73, 108.39, 30.98.

### 2. Preparation of cell

An HCT116 cell (human colon adenocarcinoma-derived cell) in which a plasmid containing an OsTIR1(WT) gene or OsTIR1(F74G) gene was introduced and an mAID-EGFP (green fluorescent protein)-NLS (nuclear localization signal) gene was inserted onto a chromosome (hereinafter, referred to as "OsTIR1(WT)/mAID-EGFP-NLS expressing cell" or "OsTIR1(F74G)/mAID-EGFP-NLS expressing cell") was prepared (refer to Fig. 1).

### 3. Addition of auxin analog

The auxin analog (concentration in culture medium: 0, 50 nM, 100 nM, 500 nM or 1 µM) was added to the OsTIR1(F74G)/mAID-EGFP-NLS expressing cell and cultured for 24 hours. In addition, as a control, a cell containing an auxin added to the OsTIR1(WT)/mAID-EGFP-NLS expressing cell was also prepared.

### 4. FACS analysis

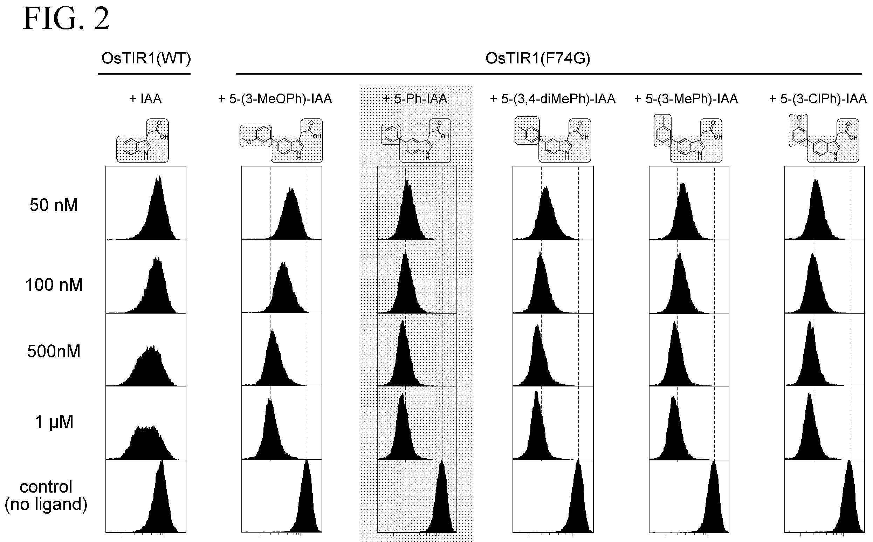

After 24 hours from the addition of each auxin analog, the cell was collected, and FACS analysis was performed. The results are shown in Fig. 2. In Fig. 2, "control" indicates cells to which only DMSO as an auxin analog solvent was added.

In the OsTIR1 (F74G)/mAID-EGFP-NLS expressing cells to which the auxin analog was added, the degradation of GFP was observed at low concentrations from Fig. 2 compared with the OsTlR1(WT)/mAID-EGFP-NLS expressing cells to which an auxin was added.

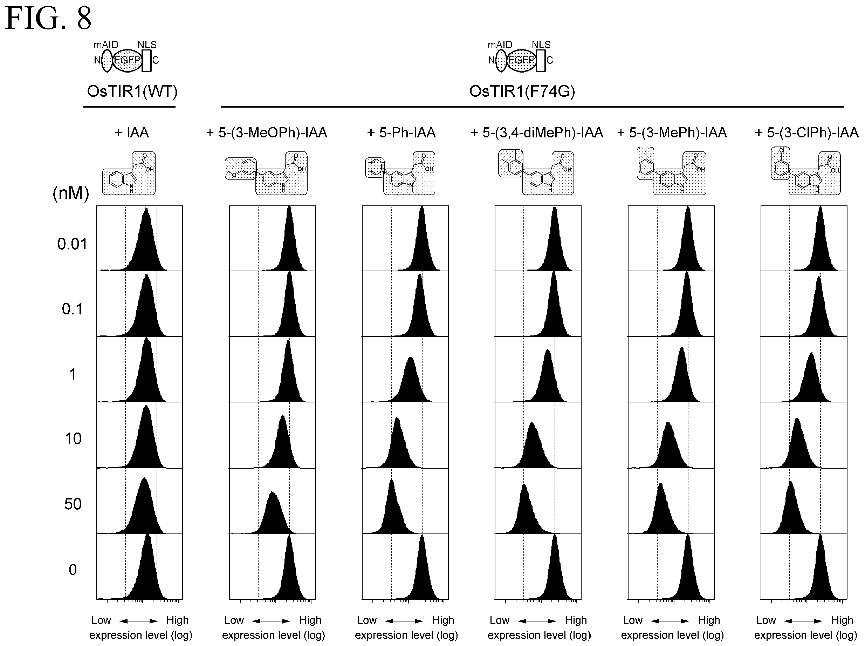

Furthermore, the OsTIR1(F74G)/mAID-EGFP-NLS expressing cell was treated with a low concentration of each auxin analog. The results are shown in Fig. 8. Among the auxin analogs used, 5-Ph-IAA showed a stronger effect of inducing the degradation of the mAID-EGFP-NLS reporter than the other compounds at 1 nM and 10 nM.

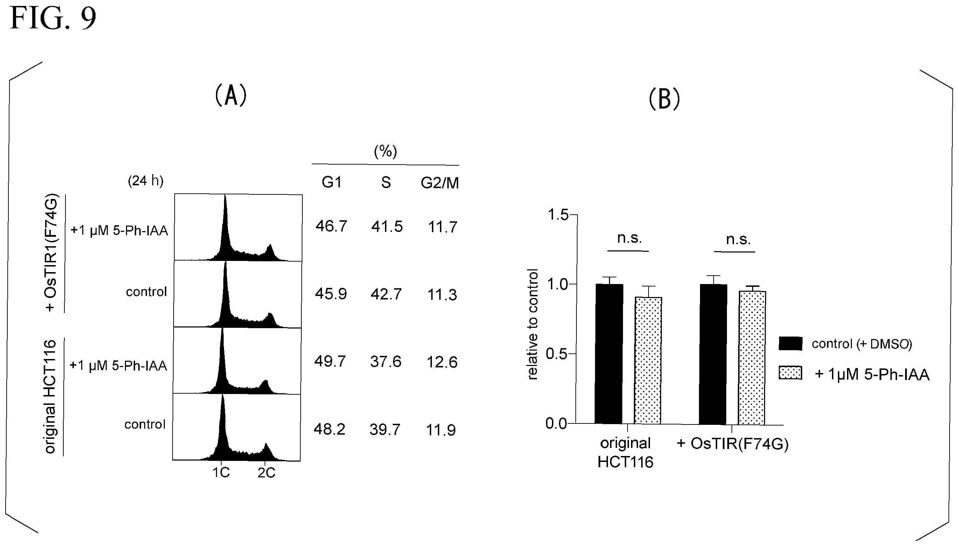

As shown in Fig. 9(A) in which the influence of the treatment with 1 µM of 5-Ph-IAA on the HCT116 cell was investigated, no influence was shown on the cell cycle. As shown in Fig. 9(B), no change in the colony formation efficiency was shown. It was confirmed that 5-Ph-IAA is an effective ligand and has no toxicity at 1 µM.

### [Example 3]

### 1. Preparation of cell

In the HCT116 cell, an OsTIR1(WT) gene or OsTIR1(F74G) gene was introduced into an AAVS1 gene locus, which is a safe harbor site, using a CRISPR/Cas system, and an OsTIR1(WT)-expressing HCT116 cell or OsTIR1(F74G)-expressing HCT116 cell was constructed.

### 2. Colony formation test

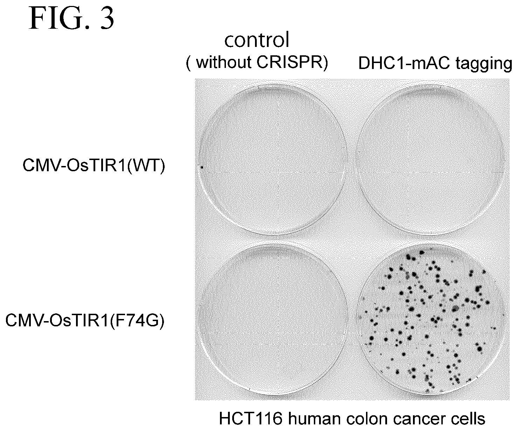

A plasmid vector having mAID, clover and a neomycin resistance gene and having a homologous sequence with respect to a DHC1 gene so as to sandwich mAID and the neomycin resistance gene was introduced into the OsTIR1(WT)-expressing HCT116 cell or OsTIR1(F74G)-expressing HCT116 cell using a CRISPR/Cas9 system, and homologous recombination with the endogenous DHC1 gene was performed. Next, after selection by G418, the formed colony was dyed using crystal violet. "DHC1" is the abbreviation for dynein heavy chain 1. The results are shown in Fig. 3.

The colony formation was not confirmed from Fig. 3 since no resistance gene works without CRISPR. In the OsTIR1(WT)-expressing HCT116 cell, the colony formation was not confirmed probably because auxin-independent degradation induction occurred even in the homologous recombinant cell. On the other hand, in the OsTIR1(F74G)-expressing HCT116 cell, the colony formation was confirmed in the homologous recombinant cell.

### 3. Degradation induction test

A degradation induction test of DHC1 was performed using the HCT116 cell expressing OsTIR1(F74G) on which homologous recombination had been performed in the above-described section 2 (hereinafter, referred to as "OsTIR1(F74G)/DHC1-mAID-Clover expressing cell").

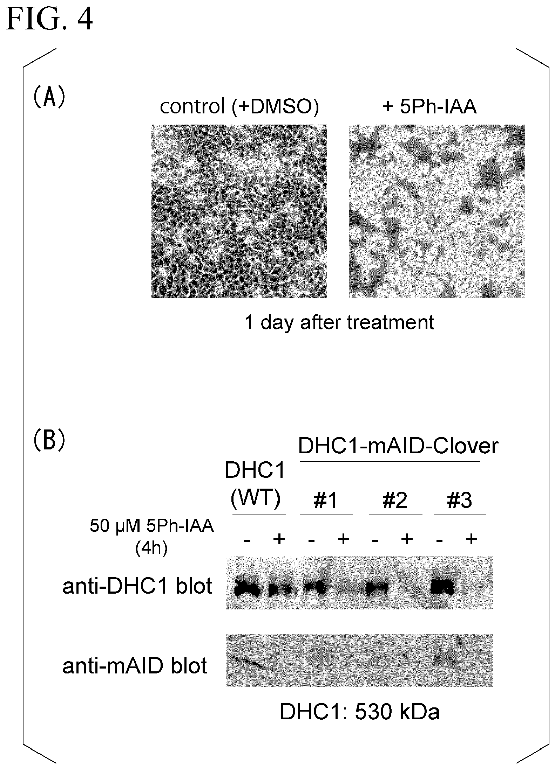

The compound indicated by the formula (I-2) (also referred to as 5-Ph-IAA) was added to the OsTIR1(F74G)/DHC1-mAID-Clover expressing cell, and the appearance of the cell after 24 hours was observed (Fig. 4(A)).

As shown in Fig. 4(A), the addition of 5-Ph-IAA degraded a DHC1-mAID-Clover fused protein, whereby accumulation of cells in the mitotic period was observed.

Furthermore, the degradation of DHC1 was confirmed by Western blotting (Fig. 4(B)). As shown in Fig. 4(B), in all of the clones #1 to #3 generated by homologous recombination, the degradation of DHC1 and mAID was confirmed in a 5-Ph-IAA-dependent manner.

### [Example 4]

In constructing an endogenous target protein degradation system, there are three difficulties described below. (1) Since a number of cultured cells become polyploids, the allele of a target protein has two or more copies. Therefore, it is difficult to construct this degradation system in a variety of cultured cell lines. (2) There is a need to establish a parent strain into which OsTIR1(F74G) has been introduced in advance. (3) It takes time and effort to construct a library of a number of degradation systems.

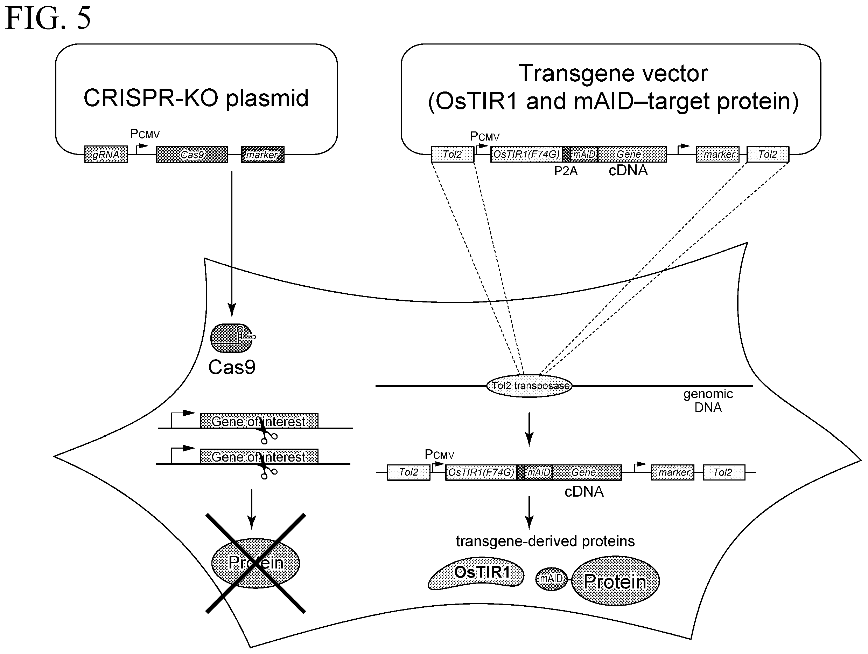

In order to solve such problems, a new target protein degradation system shown in Fig. 5 was constructed. In this degradation system, (1) a transposon vector in which an OsTIR1(F74G) gene and an mAID-target gene are connected by a P2A linker gene and (2) a CRISPR-KO vector for knocking out an endogenous target gene containing gRNA and a Cas9 gene are contained.

In this degradation system, the OsTIR1(F74G) gene and the mAID-target gene in a genome are integrated by the transposon vector, and an endogenous target gene is knocked out by the CRISPR-KO vector.

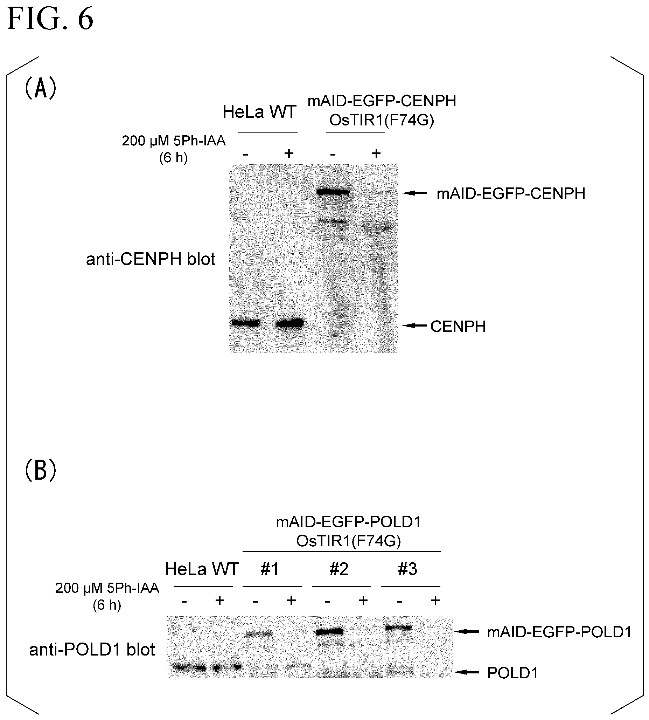

The transposon vector in which an OsTIR1(F74G) gene and an mAID-EGFP-CENPH of mAID-EGFP-POLD1 target gene were connected by a P2A linker gene was constructed, and this vector was introduced into a HeLa cell together with the CRISPR-KO vector targeting an endogenous CENPH or POLD1 gene. 5-Ph-IAA was added to such a cell, and the degradation of the target protein was confirmed by Western blotting (Fig. 6).

Fig. 6(A) shows the result of the Western blotting in which an anti-CENPH antibody was used. It was confirmed that the endogenous CENPH gene was knocked out, and the degradation of mAID-EGFP-CENPH was confirmed in a 5-Ph-IAA-dependent manner.

Fig. 6(B) shows the result of the Western blotting in which an anti-POLD1 antibody was used. It was confirmed that, in the clones #1 to #3, the endogenous POLD1 gene was knocked out, and the degradation of mAID-EGFP-POLD1 was confirmed in a 5-Ph-IAA-dependent manner.

### [Example 5]

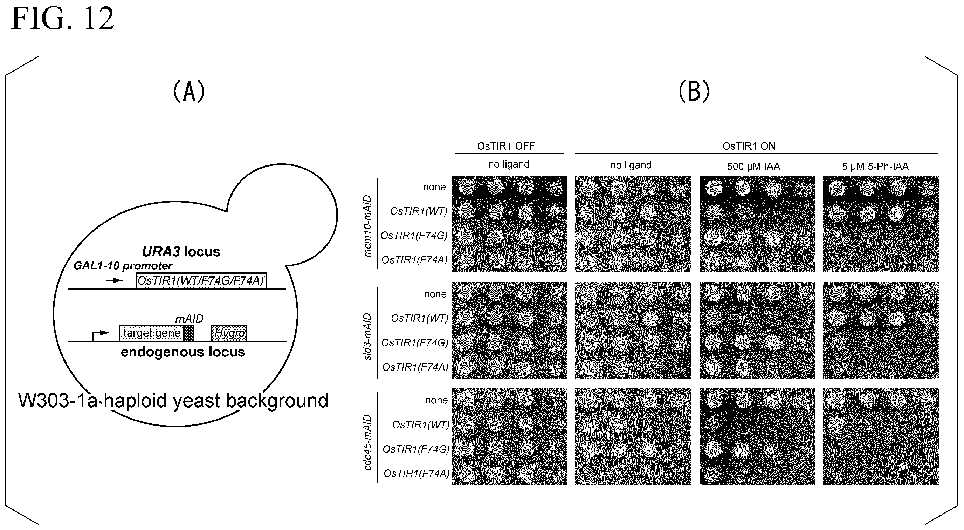

In order to confirm that the AID2 system functions even in yeasts, OsTIR1(WT, F74G or F74A) was introduced into a URA3 gene locus under the control of a GAL1-10 promoter (refer to Fig. 12(A)). After that, an mAID tag was attached to a gene that encodes MCM10, SLD3 or CDC45, which is an essential replication initiator.

For example, as shown in OsT1R1 ON with no ligand, cdc45-mAID and the like in Fig. 12(B), yeast strains expressing OsTIR1(WT or F74A) showed slow growth, and it was suggested that higher basal degradation occurred in yeasts.

Furthermore, the fact that, in OsTIR1(WT and F74A), the growth suppression of these mAID-introduced strains was observed with 500 µM of IAA suggests that OsTIR1(F74A) was still reactive to IAA.

In all cases, strains expressing OsTIR1(F74G or F74A) showed strong growth inhibition on plates containing 5 µM of 5-Ph-IAA. In a case where OsT1R1(F74G or F74A) was expressed, the growth of mem10-mAID strains and sld3-mAID strains was strongly inhibited, showing that the AID2 system has a higher degradation capability and is capable of producing mutant organisms having a strong phenotype.

### [Example 6]

A human HCT116 cell constantly expressing OsTIR1 (F74G) was used as a material, an RAD21-mAID-Clover (RAD21-mAC) cell was generated by genome editing, and an effect of the addition of 5-Ph-IAA was confirmed. As shown in Figs. 13(A) and (B), the expression level of RAD21-mAC was higher in a cell expressing OsTIR1 (F74G) than in a cell expressing OsTIR1(WT) at 0 minutes before addition, and basal degradation was shown in conventional AID systems.

RAD21-mAC rapidly disappeared after the addition of a ligand in both cases of OsTIR1(WT) and OsTIR1(F74G). Here, T1/2 was 26.5 minutes and 11.7 minutes in OsTIR1(WT) and OsTIR1(F74G), respectively, and it was shown that RAD21-mAC was more rapidly degraded by the AID2 system. These results show that the AID2 system is capable of more rapidly controlling RAD21-mAC than conventional AID systems.

As a result of investigating the cell cycle in order to view the phenotype of the rapid RAD21-mAC degradation by the AID2 system, it was confirmed that the majority of the RAD21-mAC degraded cells were arrested in the G2/M phase (refer to Fig. 13(C)). The RAD21-mAC degraded cell coincided with the essential role of RAD21 and showed a profpund defect in sister chromatidcohesion (refer to Fig. 13(D)).

### [Example 7]

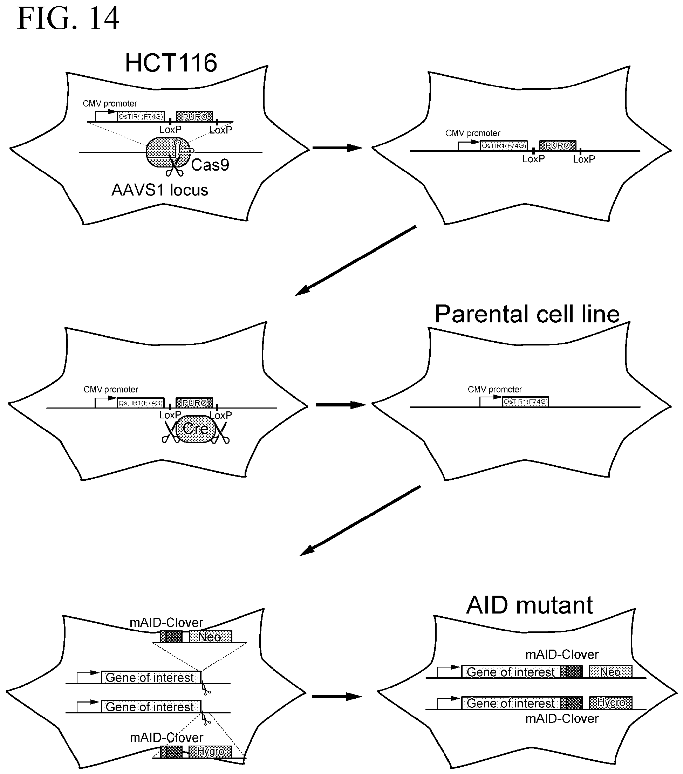

In consideration of the fact that OsTIR1 (F74G) shows lower basal degradation, whether or not DHC1-mAID-Clover (DHC1-mAC) can be generated in a HCT116 cell constantly expressing OsTIR1(F74G) was examined. A CRISPR plasmid for adding a degron tag and two donors each having a neomycin-resistant marker or a hygromycin-resistant marker were transfected into a parent cell line constitutively expressing OsTIR1(WT or F74G) (refer to Fig. 14). In an OsTIR1(F74G) expressing cell, a colony was formed in the presence of G418 and hygromycin, and tags were added to DHC1 genes at both alleles (refer to Fig. 15(A)). In a cell expressing OsTIR1(WT), no colony was formed. In a clone expressing OsTIR1(F74G), DHC1-mAC was more efficiently degraded by the addition of 1 µM of 5-Ph-IAA (refer to Fig. 15(B)). These cells were arrested in mitosis (refer to Fig. 15C) and showed a strong defect in mitotic spindle formation (refer to Fig. 15(D)). This coincides with the fact that DHC1 is essential for the formation of a spindle that is formed during mitosis.

Furthermore, whether or not a DHC1-mAC mutant organism can be generated using AtAFB2, which is a TIR1 paralog of arabidopsis, was examined. As shown in Fig. 15(A), it was possible to obtain colonies, but the number of the obtained colonies was small compared with that of OsTIR1(F74G).

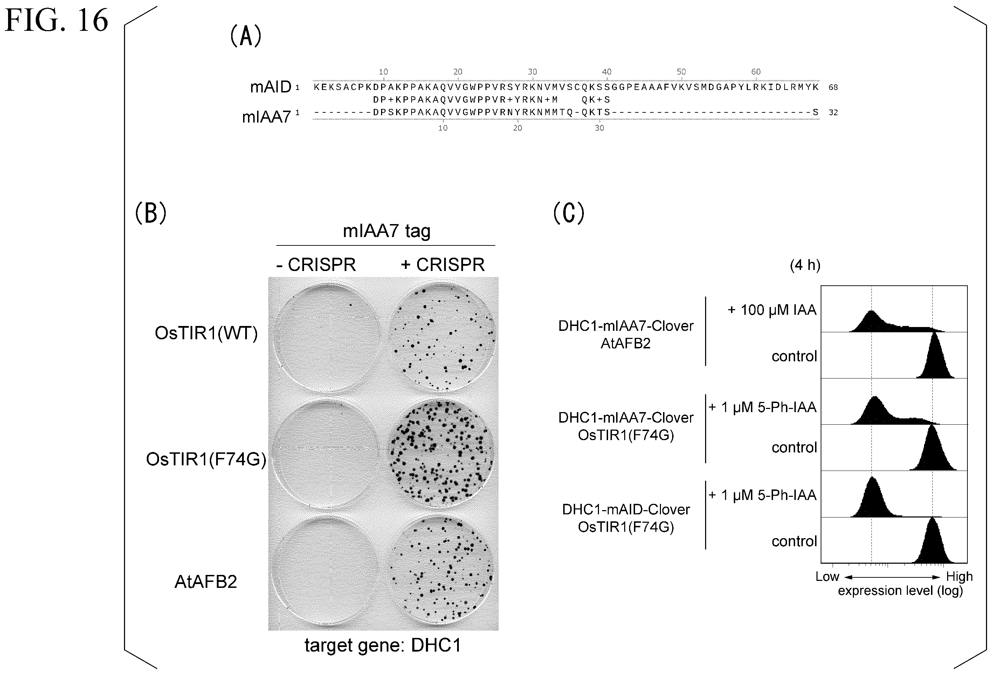

Furthermore, the same experiment was performed using, instead of mAID, mini-IAA7 (mIAA7) as another degron (refer to Fig. 16). As shown in Fig. 16(B), it was confirmed that a colony was formed under all conditions. Here, the number of colonies was small in cells expressing OsTIR1(WT) and AtAFB2 compared with the cell expressing OsTIR1(F74G).

In the cells expressing AtAFB2 or OsTIR1(F74G), the degradation of mIAA7 or mAID tag-attached DHC1 was induced (refer to Fig. 16(C)). In Fig. 16(C), it was confirmed that a combination of OsTIR1 (F74G) and an mAID tag was most efficient. This is considered to be because, as shown in Fig. 16(A), the length of mIAA7 was short and/or the number of lysine residues necessary for the ubiquitination of mIAA7 was small and thus the efficiency of ternary complex formation by IAA dropped.

### [Example 8]

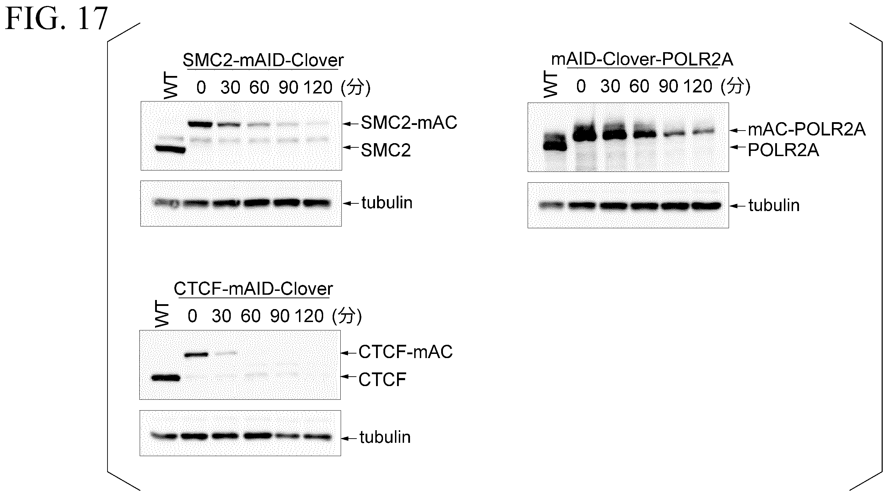

Regarding aSMC2 condensin complex, a CTCF insulator protein or the maximum subunit of RNA polymerase 2 (POLR2A) in which it was difficult to produce an AID cell line using an HCT116 cell constantly expressing OsTIR1(WT), whether or not a cell line can be generated with a cell constitutively expressing OsTIR1(F74G) was examined. As shown in Fig. 17, it was confirmed that it was possible to attach tags to both alleles of endogenous SMC2, CTCF and POLR2A in the HCT116 constitutively expressing OsTIR1(F74G). In these cell lines, mAID fused targets were rapidly degraded by the addition of 1 µM of 5-Ph-IAA. From these results, it was confirmed that the AID2 system in which a cell constitutively expressing OsTIR1(F74G) is used makes it possible to produce genes from which it is not possible to produce a cell line using former AID systems.

### [Industrial Applicability]

According to the auxin-inducible degron system of the present invention, since it is possible to inhibit auxin-independent target protein degradation, strict and full control of target protein degradation is possible.

## Claims

1. A target protein degradation method using an auxin-inducible degron system that controls degradation of a target protein in a mammalian cell, the method comprising:
culturing the mammalian cell, wherein the mammalian cell comprises
a first nucleic acid that encodes a mutant TIR1 family protein having a mutation at an auxin-binding site;
and
a second nucleic acid that encodes a degradation tag including at least a part of an Aux/IAA family protein and having an affinity to a complex of the mutant TIR1 family protein and an auxin analog,
and adding an effective concentration of 10 nM to 1µM of the auxin analog to the culture medium,
wherein the mutant TIR1 family protein is a OsTIR1 protein of which an amino acid sequence is at least 80% identical to SEQ ID NO:3 and in which an F74 of the OsTIR1 has mutated into A, G or S,
and wherein the auxin analog is a compound of the following general formula (I-1) or an ester body thereof, 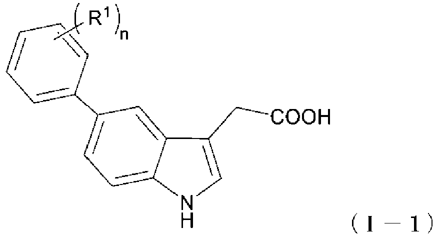 wherein in the general formula (I-1), R¹ is an alkyl group having 1 to 6 carbon atoms, an alkoxy group having 1 to 6 carbon atoms, or a halogen atom. n is an integer of 0 to 5, and, in a case where n is an integer of 2, n R1s are identical to or different from each other, and wherein the alkyl group or the alkyl group of the alkoxy group is a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a neopentyl group, a tert-pentyl group, a 2,3-dimethylpropyl group, a 1-ethylpropyl group, a 1-methylbutyl group, a 2-methylbutyl group, an n-hexyl group, an isohexyl group, a 2-methylpentyl group, a 3-methylpentyl group, a 1,1,2-trimethylpropyl group, or a 3,3-dimethylbutyl group.

2. The method according to Claim 1, wherein the cell further comprises
a third nucleic acid that encodes the target protein linked upstream or downstream of the second nucleic acid.

3. The method according to Claim 1 or 2, wherein the cell further comprises
a fourth nucleic acid that encodes a linker for controlling a plurality of genes with one promoter and that is linked between the first nucleic acid, and the second nucleic acid and the third nucleic acid.

4. The method according to any one of Claims 1 to 3, wherein the cell further comprises
a transposon vector containing the first nucleic acid and/or the second nucleic acid and the third nucleic acid.

5. The method according to any one of Claims 1 to 4, wherein the degradation tag is mAID of which the amino acid sequence is SEQ ID NO:8.

## Patentansprüche

1. Verfahren zum Abbau von Zielprotein unter Verwendung eines auxininduzierbaren Degronsystems, das Abbau eines Zielproteins in einer Säugetierzelle steuert, wobei das Verfahren Folgendes umfasst:
Kultivieren der Säugetierzelle, wobei die Säugetierzelle Folgendes umfasst
eine erste Nukleinsäure, die ein mutiertes Protein der TIR1-Familie kodiert und eine Mutation an einer Auxin-Bindungsstelle aufweist;
und
eine zweite Nukleinsäure, die einen Abbau-Tag kodiert, mindestens einen Teil eines Proteins aus der Aux/IAA-Familie einschließt und eine Affinität zu einem Komplex aus dem mutierten Protein aus der TIR1-Familie und einem Auxin-Analogon aufweist,
und Hinzufügen einer wirksamen Konzentration von 10 nM bis 1 µM des Auxin-Analogons zu dem Kulturmedium,
wobei das mutierte Protein der TIR1-Familie ein OsTIR1-Protein ist, dessen Aminosäuresequenz zu mindestens 80 % mit SEQ ID NO:3 identisch ist und in dem ein F74 des OsTIR1 zu A, G oder S mutiert ist,
und wobei das Auxin-Analogon eine Verbindung der folgenden allgemeinen Formel (I-1) oder ein Esterkörper davon ist, 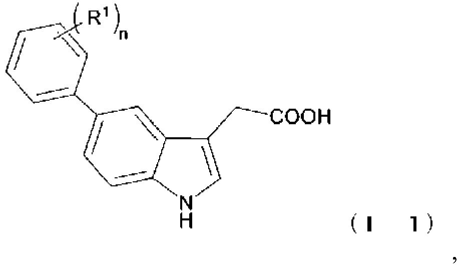
wobei in der allgemeinen Formel (I-1) R¹ eine Alkylgruppe mit 1 bis 6 Kohlenstoffatomen, eine Alkoxygruppe mit 1 bis 6 Kohlenstoffatomen oder ein Halogenatom ist, n eine ganze Zahl von 0 bis 5 ist, und in einem Fall, in dem n eine ganze Zahl 2 ist, n R1 gleich oder verschieden voneinander sind, und wobei die Alkylgruppe oder die Alkylgruppe der Alkoxygruppe eine Methylgruppe, eine Ethylgruppe, eine n-Propylgruppe, eine Isopropylgruppe, eine n-Butylgruppe, eine Isobutylgruppe, eine sec-Butylgruppe, eine tert-Butylgruppe, eine n-Pentylgruppe, eine Isopentylgruppe, eine sec-Pentylgruppe, eine Neopentylgruppe, eine tert-Pentylgruppe, eine 2,3-Dimethylpropylgruppe, eine 1-Ethylpropylgruppe, eine 1-Methylbutylgruppe, eine 2-Methylbutylgruppe, eine n-Hexylgruppe, eine Isohexylgruppe, eine 2-Methylpentylgruppe, eine 3-Methylpentylgruppe, eine 1,1,2-Trimethylpropylgruppe oder eine 3,3-Dimethylbutylgruppe ist.

2. Verfahren nach Anspruch 1, wobei die Zelle ferner Folgendes umfasst
eine dritte Nukleinsäure, die das Zielprotein kodiert und die stromaufwärts oder stromabwärts der zweiten Nukleinsäure verknüpft ist.

3. Verfahren nach Anspruch 1 oder 2, wobei die Zelle ferner Folgendes umfasst
eine vierte Nukleinsäure, die einen Linker zum Steuern einer Vielzahl von Genen mit einem Promotor kodiert und die zwischen der ersten Nukleinsäure und der zweiten Nukleinsäure und der dritten Nukleinsäure verknüpft ist.

4. Verfahren nach einem der Ansprüche 1 bis 3, wobei die Zelle ferner Folgendes umfasst
einen Transposonvektor, der die erste Nukleinsäure und/oder die zweite Nukleinsäure und die dritte Nukleinsäure enthält.

5. Verfahren nach einem der Ansprüche 1 bis 4, wobei der Abbau-Tag mAID ist, dessen Aminosäuresequenz SEQ ID NO:8 ist.

## Revendications

1. Procédé de dégradation de protéine cible à l'aide d'un système de dégron inductible par l'auxine qui contrôle la dégradation d'une protéine cible dans une cellule de mammifère, le procédé comprenant :
la culture de la cellule de mammifère, dans lequel la cellule de mammifère comprend
un premier acide nucléique qui code pour une protéine mutante de la famille TIR1 présentant une mutation au niveau d'un site de liaison à l'auxine ;
et
un deuxième acide nucléique qui code pour une étiquette de dégradation comprenant au moins une partie d'une protéine de la famille Aux/IAA et présentant une affinité pour un complexe de la protéine mutante de la famille TIR1 et d'un analogue d'auxine,
et l'ajout d'une concentration efficace de 10 nM à 1 µM de l'analogue d'auxine au milieu de culture,
dans lequel la protéine mutante de la famille TIR1 est une protéine OsTIR1 dont une séquence d'acides aminés est identique à au moins 80 % à la SEQ ID N °: 3 et dans laquelle un F74 de l'OsTIR1 a muté en A, G ou S,
et dans lequel l'analogue d'auxine est un composé de la formule générale (I-1) suivante ou un corps ester de celui-ci, 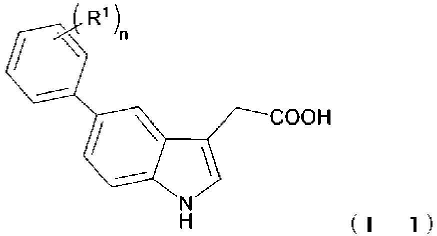
dans lequel, dans la formule générale (I-1), R¹ est un groupe alkyle comportant 1 à 6 atomes de carbone, un groupe alcoxy comportant 1 à 6 atomes de carbone, ou un atome d'halogène, n est un entier de 0 à 5 et, dans le cas où n est un entier de 2, les n R¹ sont identiques ou différents les uns des autres, et dans lequel le groupe alkyle ou le groupe alkyle du groupe alcoxy est un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe néopentyle, un groupe tert-pentyle, un groupe 2,3-diméthylpropyle, un groupe 1-éthylpropyle, un groupe 1-méthylbutyle, un groupe 2-méthylbutyle, un groupe n-hexyle, un groupe isohexyle, un groupe 2-méthylpentyle, un groupe 3-méthylpentyle, un groupe 1,1,2-triméthylpropyle ou un groupe 3,3-diméthylbutyle.

2. Procédé selon la revendication 1, dans lequel la cellule comprend en outre
un troisième acide nucléique qui code pour la protéine cible liée en amont ou en aval du deuxième acide nucléique.

3. Procédé selon l'une des revendications 1 ou 2, dans lequel la cellule comprend en outre
un quatrième acide nucléique qui code pour un lieur destiné à contrôler une pluralité de gènes avec un promoteur et qui est lié entre le premier acide nucléique, et le deuxième acide nucléique et le troisième acide nucléique.

4. Procédé selon l'une quelconque des revendications 1 à 3, dans lequel la cellule comprend en outre
un vecteur transposon contenant le premier acide nucléique et/ou le deuxième acide nucléique et le troisième acide nucléique.

5. Procédé selon l'une quelconque des revendications 1 à 4, dans lequel l'étiquette de dégradation est mAID dont la séquence d'acides aminés est SEQ ID N° : 8.
